# FASCICULE DE BREVET EUROPEEN

(11) **EP 3 394 016 B1**
(45) Date de publication et mention de la délivrance du brevet: **10.05.2023**
(21) Numéro de dépôt: 16809419.1
(22) Date de dépôt: 14.12.2016
(51) Int. Cl.: C07C 17/25, C07C 17/38, C07C 17/386, C07C 21/18, C09K 5/04

(54) **PROCEDE DE PRODUCTION ET DE PURIFICATION DU 2,3,3,3-TETRAFLUORO-1-PROPENE**
VERFAHREN ZUR HERSTELLUNG UND REINIGUNG VON 2,3,3,3-TETRAFLUOR-1-PROPEN
METHOD FOR PRODUCING AND PURIFYING 2,3,3,3-TETRAFLUORO-1-PROPENE

(30) Priorité: 23.12.2015 FR 1563165
(43) Date de publication de la demande: 31.10.2018
(73) Titulaire: Arkema France, 92700 Colombes (FR)
(72) Inventeur: BABA-AHMED, Abdelatif, 69190 Saint-Fons (FR); COLLIER, Bertrand, 69230 Saint-Genis-Laval (FR); DEUR-BERT, Dominique, 69390 Charly (FR); WENDLINGER, Laurent, 69510 Soucieu en Jarrest (FR)
(74) Mandataire: Arkema Patent
(86) Numéro de dépôt international: PCT/EP2016/080945
(87) Numéro de publication internationale: WO 2017/108519

(56) Documents cités:
- EP-A1- 0 921 109
- EP-A1- 2 671 860
- EP-A1- 2 939 994
- EP-B1- 0 743 934
- WO-A1-2014/147310
- WO-A1-2014/147311
- WO-A1-2014/147314

## Description

### Domaine technique de l'invention

L'invention se rapporte à un procédé de purification du 2,3,3,3-tetrafluoro-1-propène. En outre, l'invention se rapporte également à un procédé de production et de purification du 2,3,3,3-tetrafluoro-1-propène.

### Arrière-plan technologique de l'invention

Les hydrofluorocarbures (HFC) et en particulier les hydrofluorooléfines (HFOs), telles que le 2,3,3,3-tétrafluoro-1-propène (HFO-1234yf) sont des composés connus pour leurs propriétés de réfrigérants et fluides caloporteurs, extinctrices, propulseurs, agents moussants, agents gonflants, diélectriques gazeux, milieu de polymérisation ou monomère, fluides supports, agents pour abrasifs, agents de séchage et fluides pour unité de production d'énergie. Les HFO ont été identifiés comme des alternatives souhaitables au HCFC du fait de leurs faibles valeurs d'ODP (Ozone Depletion Potential ou potentiel d'appauvrissement de la couche d'ozone) et de GWP (Global Warming Potential ou potentiel de réchauffement climatique).

La plupart des procédés de fabrication des hydrofluorooléfines font appel à une réaction de fluoration et / ou déhydrohalogénation. Ces réactions sont effectuées en phase gazeuse et génèrent des impuretés qu'il faut par conséquent éliminer pour obtenir le composé désiré dans un degré de pureté suffisant pour les applications visées.

Par exemple, dans le cadre de la production de 2,3,3,3-tétrafluoro-1-propène (HFO-1234yf), la présence d'impuretés telles que le 1-chloro-3,3,3-trifluoro-1-propene (1233zd), 1,3,3,3-tetrafluoro-1-propene (1234ze) et le 1,1,1,3,3-pentafluoropropane (245fa) sont observées. Ces impuretés sont des isomères des composés principaux visant à être obtenues par le procédé de production du 2,3,3,3-tétrafluoro-1-propène outre ce dernier, i.e. 2-chloro-3,3,3-trifluoro-1-propene (1233xf) et 1,1,1,2,2-pentafluoropropane (245cb). Compte tenu des points d'ébullition respectifs du 1-chloro-3,3,3-trifluoro-1-propene (1233zd), 1,3,3,3-tetrafluoro-1-propene (1234ze) et le 1,1,1,3,3-pentafluoropropane (245fa), ceux-ci peuvent s'accumuler dans le réacteur et ainsi empêcher la formation des produits d'intérêt.

La purification de ce type de mélange réactionnel peut être effectuée par différentes techniques connues de l'art antérieur, telles que par exemple la distillation. Cependant lorsque les composés à purifier ont des points d'ébullition trop proche ou lorsque ceux-ci forment des compositions azéotropes ou quasi-azéotropes, la distillation n'est pas un procédé efficace. Des procédés de distillation extractive ont ainsi été décrits.

On connaît par EP 0 864 554 un procédé de purification d'un mélange comprenant 1,1,1,3,3-pentafluoropropane (245fa) et 1-chloro-3,3,3-trifluoro-trans-1-propene (1233zd) par distillation en présence d'un solvant ayant un point d'ébullition supérieur à celui de 1-chloro-3,3,3-trifluoro-trans-1-propene.

On connaît par WO 03/068716 un procédé de récupération de pentafluoroéthane à partir d'un mélange comprenant du pentafluoroéthane et du chloropentafluoroéthane par distillation en présence d'hexafluoropropène.

On connaît aussi par WO 98/19982 un procédé de purification du 1,1-difluoroéthane par distillation extractive. Le procédé consiste à mettre en contact un agent d'extraction avec un mélange de 1,1-difluoroéthane et de chlorure de vinyle. L'agent d'extraction est choisi parmi les hydrocarbures, les alcools, les chlorocarbures ayant un point d'ébullition compris entre 10°C et 120°C.

On connaît aussi par EP 2 718 860 un procédé de purification du R-1234yf par mise en contact avec un solvant ayant un index d'extraction d'au moins 6,5.

Comme mentionné par WO 98/19982, la sélection de l'agent d'extraction peut s'avérer complexe en fonction des produits à séparer. Il existe donc toujours un besoin pour la mise en oeuvre d'un procédé particulier de purification du 2,3,3,3-tetrafluoro-1-propène.

### Résumé de l'invention

Dans un procédé de production du 2,3,3,3-tetrafluoro-1-propène, le choix de conditions opérationnelles particulières peut favoriser la présence de certaines impuretés ou d'isomères de celles-ci. La présence d'impuretés telles que 1,3,3,3-tetrafluoro-1-propene (1234ze) peut être observée tout comme celle de 1-chloro-3,3,3-trifluoro-1-propene (1233zd), et 1,1,1,3,3-pentafluoropropane (245fa). Ces impuretés peuvent provenir de réactions secondaires induites par des composés produits intermédiairement pendant la production du 2,3,3,3-tetrafluoro-1-propène, et peuvent présenter des propriétés physiques telles que leur élimination peut s'avérer complexe. La présente invention permet la production de 2,3,3,3-tetrafluoro-1-propène avec une pureté améliorée.

Selon un premier aspect, la présente invention fournit un procédé de purification du 2,3,3,3-tétrafluoro-1-propène (1234yf) à partir d'une première composition comprenant du 2,3,3,3-tétrafluoro-1-propène, 3,3,3-trifluoropropene (1243zf), trans-1,3,3,3-tetrafluoro-1-propene (1234ze-E) et optionnellement ou non au moins un composé sélectionné parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), ledit procédé comprenant les étapes de:
a) mise en contact de ladite première composition avec au moins un agent d'extraction organique pour former une seconde composition ;
b) distillation extractive de ladite seconde composition pour former :
   i) une troisième composition comprenant ledit agent d'extraction organique, 3,3,3-trifluoropropene (1243zf), trans-1,3,3,3-tetrafluoro-1-propene (1234ze-E) et optionnellement ou non au moins un composé sélectionné parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E); et
   ii) un courant comprenant le 2,3,3,3-tétrafluoro-1-propène,
c) récupération et séparation de ladite troisième composition pour former un courant comprenant ledit agent d'extraction organique et un courant comprenant 3,3,3-trifluoropropene (1243zf), trans-1,3,3,3-tetrafluoro-1-propene (1234ze-E) et optionnellement ou non au moins un composé sélectionné parmi le groupe consistant en chlorométhane (40), 1,1-difluoroéthane (152a), chloropentafluoroéthane (115), 1,1,1,2-tetrafluoroethane (134a), et trans-1,2,3,3,3-pentafluoropropene (1225ye-E) ; de préférence le courant comprenant ledit agent d'extraction organique est recyclé à l'étape a) ;
caractérisé en ce que ledit agent d'extraction organique est choisi parmi le groupe consistant en acetaldehyde, methylformate, 2-methoxy-1-propene, 1,2-epoxypropane, ethoxy-ethene, dimethoxymethane, 1-chloro-1,2,2-trifluoropropane, 3-chloro-1,1,1-trifluoropropane, ethanedial, 2-chloro-1,1,1,3-tetrafluoropropane, propanone, methylacetate, 4-methoxy-2-methyl-2-butanethiol, difluorodiethylsilane, isobutanal, isopropylformate, methylglyoxal, 2,3-dichloro-1,1,1-trifluoropropane, ethylacetate, butanone, n-propylformate, 1,3-dichloro-1,1,2-trifluoropropane, 1,1-dichloro-2,2,3-trifluoropropane, 1,2-dimethoxyethane, 1,3-dichloro-1,2,2-trifluoropropane, isopropylacetate, diethylsulfide, 1,3-dichloro-1,2,3-trifluoropropane, 2-methylbutanal, 2-allyloxyethanol, ethylpropionate, 1,2-dimethoxypropane, dioxane, 3-pentanone, 2-pentanone, trimethoxymethane, 1,3-dioxane, 3,3-dimethyl-2-butanone, pyridine, n-methylmorpholine, 4-methyl-2-pentanone, butyronitrile, 1-methoxy2-propanol, diethylcarbonate, n-butylacetate, 2-hexanone, 5-hexen-2-one, 2-methoxy1-propanol, 1-ethoxy-2-propanol, hexanal, 2-(dimethylamino)-ethanol, 2-methylpyrazine, 2-ethoxy-1-propanol, 1-methylpiperazine, n-ethyl-morpholine, valeronitrile, 4-methyl-2-hexanone, 1-methoxy-2-acetoxypropane, 2,6-dimethylmorpholine, methylhexanoate, 2-propoxyethanol, 1-propoxy-2-propanol.

De préférence, le courant comprenant le 2,3,3,3-tétrafluoro-1-propène formé à l'étape b) est récupéré. Ce courant peut être éventuellement purifié pour atteindre un degré de pureté adapté à des spécifications commerciales particulières.

Selon un mode de réalisation préféré, ladite première composition est une composition azéotropique ou quasi-azéotropique comprenant 2,3,3,3-tetrafluoro-1-propène, trans-1,3,3,3-tetrafluoro-1-propene (1234ze-E), 3,3,3-trifluoropropene (1243zf) et optionnellement ou non au moins un des composés sélectionné parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E).

Selon un mode de réalisation préféré, ledit agent d'extraction organique est choisi parmi le groupe consistant en ledit agent d'extraction organique est choisi parmi le groupe consistant en 2-methoxy-1-propene, 1,2-epoxypropane, ethoxy-ethene, dimethoxymethane, methylacetate, isobutanal, isopropylformate, ethylacetate, butanone, n-propylformate, 1,2-dimethoxyethane, isopropylacetate, 2-methylbutanal, ethylpropionate, 1,2-dimethoxypropane, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 2-hexanone, 5-hexen-2-one, 1-ethoxy-2-propanol, hexanal, 2-(dimethylamino)-ethanol, 2-methylpyrazine, 1-methylpiperazine, valeronitrile, 4-methyl-2-hexanone, 1-methoxy-2-acetoxypropane, 2,6-dimethylmorpholine, methylhexanoate, 1-propoxy-2-propanol ; avantageusement ledit agent d'extraction organique est choisi parmi le groupe consistant en éthoxy-ethene, dimethoxymethane, methylacetate, isobutanal, isopropylformate, ethylacetate, butanone, 1,2-dimethoxyethane, isopropylacetate, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 1-ethoxy-2-propanol, hexanal ; de préférence ledit agent d'extraction organique est choisi parmi le groupe consistant en diméthoxymethane, butanone, isopropylacetate, dioxane, trimethoxymethane, 1,3-dioxane, n-butylacetate, 1-ethoxy-2-propanol, hexanal ; en particulier ledit agent d'extraction organique est choisi parmi le groupe consistant en diméthoxymethane, isopropylacetate, dioxane, trimethoxymethane, 1,3-dioxane, n-butylacetate, 1-ethoxy-2-propanol, hexanal.

Selon un mode de réalisation préféré, le courant comprenant le 2,3,3,3-tetrafluoro-1-propène formé à l'étape b) est récupéré et est dépourvu de trans-1,3,3,3-tetrafluoro-1-propene (1234ze-E) et/ou de 3,3,3-trifluoropropene (1243zf) et/ou d'au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E).

Selon un mode de réalisation préféré, ladite première composition comprend des impuretés ayant un point d'ébullition inférieur au point d'ébullition du 2,3,3,3-tetrafluoro-1-propène et optionnellement des impuretés lourdes, et le procédé comprend préalablement à l'étape a) les étapes :
i') mise en oeuvre ou fourniture d'une composition comprenant 2,3,3,3-tetrafluoro-1-propène, des impuretés ayant un point d'ébullition inférieur au point d'ébullition du 2,3,3,3-tetrafluoro-1-propène, 3,3,3-trifluoropropene (1243zf), trans-1,3,3,3-tetrafluoro-1-propene (1234ze-E),et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), et optionnellement des impuretés lourdes ;
ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, 3,3,3-trifluoropropene (1243zf), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), et optionnellement des impuretés lourdes ; récupéré en bas de colonne de distillation ;
iii') optionnellement, distillation dudit premier courant récupéré en bas de colonne de distillation à l'étape ii') pour récupérer en tête de colonne un second courant comprenant du 2,3,3,3-tetrafluoro-1-propène, 3,3,3-trifluoropropene (1243zf), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), et en bas de colonne de distillation un courant comprenant les impuretés lourdes ;
ledit premier courant récupéré à l'étape ii') ou ledit second courant récupéré à l'étape iii') correspondent à ladite première composition mise en oeuvre à l'étape a).

Les impuretés lourdes peuvent contenir par exemple 1,1,1,3,3,3-hexafluoropropane (236fa), 1,1,1,2,3,3-hexafluoropropane (236ea), 1,1,1,2,3,3,3-heptafluoropropane (227ca), cis-1,3,3,3-tetrafluoro-1-propene (1234ze-Z), des dimères ou des trimères issus de l'un des composés présents dans la composition ou le courant considéré, par exemple des hydrocarbures en C4 ou C5 tels que hexafluorobutène (1336), heptafluorobutène (1327), octafluorobutane (338), nonafluoropentène (1429), heptafluoropentène (1447).

Selon un second aspect de la présente invention, un procédé de production de 2,3,3,3-tetrafluoro-1-propène est fourni. Ledit procédé comprend les étapes de :
A) fluoration en présence d'un catalyseur d'un composé de formule CX(Y)₂-CX(Y)ₘ-CHₘXY (I) dans laquelle X et Y représentent indépendamment H, F, ou Cl et m = 0 ou 1 ;et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;
B) récupération d'un courant comprenant du 2,3,3,3-tetrafluoro-1-propène, 3,3,3-trifluoropropene (1243zf), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), ;
C) mise en oeuvre du procédé selon l'une quelconque des revendications 1 à 5 à partir du courant récupéré à l'étape B).

Selon un autre aspect, la présente invention fournit une composition comprenant du 2,3,3,3-tetrafluoropropène, 3,3,3-trifluoropropene (1243zf), trans-1,3,3,3-tetrafluoro-1-propene (1234ze-E) et un agent d'extraction organique sélectionné parmi le groupe consistant en acétaldehyde, methylformate, 2-methoxy-1-propene, 1,2-epoxypropane, ethoxy-ethene, dimethoxymethane, 1-chloro-1,2,2-trifluoropropane, 3-chloro-1,1,1-trifluoropropane, ethanedial, 2-chloro-1,1,1,3-tetrafluoropropane, propanone, methylacetate, 4-methoxy-2-methyl-2-butanethiol, difluorodiethylsilane, isobutanal, isopropylformate, methylglyoxal, 2,3-dichloro-1,1,1-trifluoropropane, ethylacetate, butanone, n-propylformate, 1,3-dichloro-1,1,2-trifluoropropane, 1,1-dichloro-2,2,3-trifluoropropane, 1,2-dimethoxyethane, 1,3-dichloro-1,2,2-trifluoropropane, isopropylacetate, diethylsulfide, 1,3-dichloro-1,2,3-trifluoropropane, 2-methylbutanal, 2-allyloxyethanol, ethylpropionate, 1,2-dimethoxypropane, dioxane, 3-pentanone, 2-pentanone, trimethoxymethane, 1,3-dioxane, 3,3-dimethyl-2-butanone, pyridine, n-methylmorpholine, 4-methyl-2-pentanone, butyronitrile, 1-methoxy2-propanol, diethylcarbonate, n-butylacetate, 2-hexanone, 5-hexen-2-one, 2-methoxy1-propanol, 1-ethoxy-2-propanol, hexanal, 2-(dimethylamino)-ethanol, 2-methylpyrazine, 2-ethoxy-1-propanol, 1-methylpiperazine, n-ethyl-morpholine, valeronitrile, 4-methyl-2-hexanone, 1-methoxy-2-acetoxypropane, 2,6-dimethylmorpholine, methylhexanoate, 2-propoxyethanol, 1-propoxy-2-propanol.

De préférence, ledit agent d'extraction organique est sélectionné parmi le groupe consistant en 2-methoxy-1-propene, 1,2-epoxypropane, ethoxy-ethene, dimethoxymethane, methylacetate, isobutanal, isopropylformate, ethylacetate, butanone, n-propylformate, 1,2-dimethoxyethane, isopropylacetate, 2-methylbutanal, ethylpropionate, 1,2-dimethoxypropane, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 2-hexanone, 5-hexen-2-one, 1-ethoxy-2-propanol, hexanal, 2-(dimethylamino)-ethanol, 2-methylpyrazine, 1-methylpiperazine, valeronitrile, 4-methyl-2-hexanone, 1-methoxy-2-acetoxypropane, 2,6-dimethylmorpholine, methylhexanoate, 1-propoxy-2-propanol.

### Brève description des figures

Les figures 1a-c représentent schématiquement un dispositif mettant en oeuvre un procédé de purification du 2-3,3,3-tetrafluoro-1-propène selon un mode de réalisation particulier de la présente invention.
La figure 2 représente schématiquement un dispositif mettant en oeuvre un procédé de production du 2,3,3,3-tetrafluoro-1-propène selon un mode de réalisation particulier de la présente invention.

### Description détaillée de l'invention

Le terme « hydrocarbure » tel qu'utilisé ici se réfère à des composés linéaires ou branchés d'alcane en C₁-C₂₀, cycloalcane en C₃-C₂₀, alcène en C₅-C₂₀, cycloalcène en C₅-C₂₀, arène en C₆-C₁₈. Par exemple, le terme alcane se réfère à des composés de formule CₙH₂ₙ₊₂ dans lequel n est compris entre 1 et 20. Le terme alcane en C₁-C₂₀ englobe par exemple le pentane, hexane, heptane, octane, nonane, décane ou des isomères de ceux-ci. Le terme alcène en C₅-C₂₀ se réfère à des composés hydrocarbonés comprenant une ou plusieurs doubles liaisons carbone-carbone et comprenant de 5 à 20 atomes de carbone. Le terme cycloalcane en C₃-C₂₀ se réfère à un cycle hydrocarboné saturé comprenant de 3 à 20 atomes de carbone. Le terme aryle en C₆-C₁₈ se réfère à des composés hydrocarbonés cycliques et aromatiques comprenant de 6 à 18 atomes de carbone. Le terme cycloalcène en C₅-C₂₀ se réfère à des composés hydrocarbonés cycliques comprenant de 5 à 20 atomes de carbone et comprenant une ou plusieurs doubles liaisons carbone-carbone.

Le terme « alkyle » désigne un radical monovalent issu d'un alcane, linéaire ou branché, comprenant de 1 à 20 atomes de carbone. Le terme « cycloalkyle » désigne un radical monovalent issu d'un cycloalcane comprenant de 3 à 20 atomes de carbone. Le terme « aryle » désigne un radical monovalent issu d'un arène comprenant de 6 à 18 atomes de carbone. Le terme « alkényle » désigne un radical monovalent de 2 à 20 atomes de carbone et au moins une double liaison carbone-carbone. Le terme « alkynyle » désigne un radical monovalent de 2 à 20 atomes de carbone et au moins une triple liaison carbone-carbone. Le terme « halogène » se réfère à un groupement -F, -Cl, -Br ou -I. Le terme « cycloalkényle » se réfère à un radical monovalent issu d'un cycloalcène comprenant de 3 à 20 atomes de carbone. Les substituants alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ peuvent être substitués ou non par un ou plusieurs substituants -OH, halogène, -NR^{a}C(O)R^{b}, -C(O)NR^{a}R^{b} -CN, -NO₂, -NR^{a}R^{b}, -OR^{a}, -SR^{a}, -CO₂R^{a}, -OC(O)OR^{a}, -OC(O)R^{a}, - C(O)H, -C(O)R^{a} dans lequel R^{a} et R^{b} sont indépendamment l'un de l'autre hydrogène, alkyle en C₁-C₂₀ non substitué, alkényle en C₂-C₂₀ non substitué, alkynyle en C₂-C₂₀ non substitué, cycloalkyle en C₃-C₂₀ non substitué, cycloalkényle en C₃-C₂₀ non substitué, aryle en C₆-C₁₈ non substitué. Dans les substituants -NR^{a}R^{b}, R^{a} et R^{b} peuvent former avec l'atome d'azote auquel ils sont rattachés un hétérocycle saturé ou insaturé, aromatique ou non, comprenant de 5 à 10 chainons.

Le terme « hydrohalocarbures » se réfère à des composés de formule R^{a}X dans laquelle R^{a} est sélectionné parmi alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ et X représente un atome de chlore, de fluor, de brome ou d'iode. Les substituants alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ peuvent être substitués ou non par un ou plusieurs substituants -OH, halogène, -NR^{a}C(O)R^{b}, -C(O)NR^{a}R^{b} -CN, -NO₂, -NR^{a}R^{b}, -OR^{a}, - SR^{a}, -CO₂R^{a}, -OC(O)OR^{a}, -OC(O)R^{a}, -C(O)H, -C(O)R^{a} dans lequel R^{a} et R^{b} sont tels que définis ci-dessus.

Le terme « alcool » se réfère à des hydrocarbures ou hydrohalocarbures tels que définis ci-dessus dans lequel au moins un atome d'hydrogène est remplacé par un groupement hydroxyle -OH.

Le terme « cétone » se réfère à des hydrocarbures comprenant au moins un ou plusieurs groupements fonctionnels carbonyle R^{C}-C(O)-R^{d} dans lequel R^{c} et R^{d} sont indépendamment l'un de l'autre un alkyle en C₁-C₂₀, alkényle en C₂-C₂₀, alkynyle en C₂-C₂₀, cycloalkyle en C₃-C₂₀, cycloalkényle en C₃-C₂₀, aryle en C₆-C₁₈ et peuvent être substitués ou non par un ou plusieurs substituants -OH, halogène, -NR^{a}C(O)R^{b}, -C(O)NR^{a}R^{b} -CN, -NO₂, -NR^{a}R^{b}, -OR^{a}, -SR^{a}, -CO₂R^{a}, - OC(O)OR^{a}, -OC(O)R^{a}, -C(O)H, -C(O)R^{a}, dans lequel R^{a} et R^{b} sont, R^{c} et R^{d} pouvant être liés entre eux pour former avec le groupement carbonyle auquel ils sont rattachés une cétone cyclique comprenant de 4 à 10 chainons, de préférence de 4 à 7 chainons. La cétone cyclique peut également comprendre une ou plusieurs doubles liaisons carbone-carbone. La cétone cyclique peut également être substituée ou non par un ou plusieurs substituants tels que définis ci-dessus.

Le terme « amine » se réfère à des hydrocarbures comprenant au moins un ou plusieurs groupements fonctionnels amine -NR^{c}R^{d} dans lequel R^{c} et R^{d} sont tels que définis ci-dessus, R^{c} et R^{d} pouvant être liés entre eux pour former avec l'atome d'azote auquel ils sont rattachés un hétérocycle aromatique ou non comprenant de 4 à 10 chainons.

Le terme « esters » se réfère à des composés de formule R^{c}-C(O)-O-R^{d} dans lequel R^{c} et R^{d} sont tels que définis ci-dessus, R^{c} et R^{d} pouvant être liés entre eux pour former avec le groupement ester un cycle comprenant de 4 à 20 atomes de carbone.

Le terme « éther » se réfère à des composés de formule R^{c}-O-R^{d} dans lequel R^{c} et R^{d} sont tels que définis ci-dessus, R^{c} et R^{d} pouvant être liés entre eux pour former avec l'atome d'oxygène auquel ils sont rattachés un hétérocycle comprenant de 4 à 20 atomes de carbone.

Le terme « aldéhyde » se réfère à des composés comprenant au moins un ou plusieurs groupements fonctionnels -C(O)-H.

Le terme « nitrile » se réfère à des composés comprenant au moins un ou plusieurs groupements fonctionnels -CN.

Le terme « carbonate » se réfère à des composés de formule R^{c}-O-C(O)-O-R^{d} dans lequel R^{c} et R^{d} sont tels que définis ci-dessus.

Le terme « thioalkyle » concerne des composés de formule R^{c}SR^{d} dans laquelle R^{c} et R^{d} sont tels que définis ci-dessus.

Le terme « amide » concerne des composés de formule R^{c}C(O)NR^{e}R^{d} dans laquelle R^{c} et R^{d} sont tels que définis ci-dessus, R^{e} étant défini comme R^{c} ; R^{c} et R^{d} pouvant être liés entre eux pour former avec le groupement amide -C(O)N- auquel ils sont rattachés une amide cyclique comprenant de 4 à 10 chainons, de préférence de 4 à 7 chainons. L'amide cyclique peut également comprendre une ou plusieurs doubles liaisons carbone-carbone. L'amide cyclique peut également être substituée ou non par un ou plusieurs substituants tels que définis ci-dessus.

Le terme « hétérocycle » désigne un cycle carboné comprenant de 4 à 10 chaînons dont au moins un des chaînons est un hétéroatome sélectionné parmi le groupe consistant en O, S, P et N. L'hétérocycle peut comprendre un ou plusieurs double liaison carbone-carbone ou une ou plusieurs double liaison carbone-hétéroatome ou une ou plusieurs double liaison hétéroatome-hétéroatome. De préférence, l'hétérocycle peut comprendre 1, 2, 3, 4 ou 5 hétéroatomes tel que défini ci-dessus. En particulier, l'hétérocycle peut comprendre 1, 2 ou 3 hétéroatomes sélectionné parmi l'oxygène, l'azote ou le soufre. De préférence, l'hétérocycle peut être un cycle carboné comprenant de 4 à 6 chaînons dont 1, 2 ou 3 chaînons sont des hétéroatomes sélectionnés parmi O ou N. L'hétérocycle peut être ou non substitué par un ou plusieurs substituants choisi parmi -OH, halogène, -NR^{a}C(O)R^{b}, -C(O)NR^{a}R^{b} -CN, -NO₂, -NR^{a}R^{b}, -OR^{a}, -SR^{a}, - CO₂R^{a}, -OC(O)OR^{a}, -OC(O)R^{a}, -C(O)H, -C(O)R^{a} dans lequel R^{a} et R^{b} sont tels que définis ci-dessus.

Le terme « composition azéotropique » désigne un mélange liquide de deux ou plusieurs composés se comportant comme une substance unique, et qui bout à température fixe en gardant une composition en phase liquide identique à celle de la phase gaz. Le terme « composition quasi-azéotropique » désigne un mélange liquide de deux ou plusieurs composés ayant un point d'ébullition constant ou qui a tendance à ne pas se fractionner lorsqu'il est soumis à l'ébullition ou l'évaporation.

Le terme « agent d'extraction organique » se réfère à un composé comportant au moins un atome de carbone.

Selon un premier aspect, l'invention se rapporte à un procédé de purification du 2,3,3,3-tetrafluoro-1-propene (1234yf) tel que défini dans les revendications.

Le procédé de purification est effectué à partir d'une première composition comprenant du 2,3,3,3-tétrafluoro-1-propène et au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf).

Ledit procédé peut comprendre les étapes de :
a) mise en contact de ladite première composition avec au moins un agent d'extraction organique pour former une seconde composition ;
b) distillation extractive de ladite seconde composition pour former :
   i) une troisième composition comprenant ledit agent d'extraction organique et au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf); et
   ii) un courant comprenant le 2,3,3,3-tétrafluoro-1-propène,
c) récupération et séparation de ladite troisième composition pour former un courant comprenant ledit agent d'extraction organique et un courant comprenant au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf).

De préférence, le courant comprenant ledit agent d'extraction organique est recyclé à l'étape a).

Avantageusement, ladite première composition peut comprendre du 2,3,3,3-tétrafluoro-1-propène et au moins deux, au moins trois, au moins quatre, au moins cinq, au moins six ou l'ensemble des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) ; et ladite troisième composition peut comprendre au moins deux, au moins trois, au moins quatre, au moins cinq, au moins six ou l'ensemble des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf).

De préférence, ladite première composition peut comprendre du 2,3,3,3-tétrafluoro-1-propène, 3,3,3-trifluoropropene (1243zf), et au moins un des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) ; et ainsi ladite troisième composition peut comprendre 3,3,3-trifluoropropene (1243zf), et au moins un des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E).

En particulier, ladite première composition peut comprendre 2,3,3,3-tétrafluoro-1-propène, 3,3,3-trifluoropropene (1243zf), trans-1,3,3,3-tetrafluoro-1-propene (1234ze-E) et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E) ; et ladite troisième composition peut comprendre 3,3,3-trifluoropropene (1243zf), trans-1,3,3,3-tetrafluoro-1-propene (1234ze-E) et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E).

Ainsi, ledit procédé peut comprendre les étapes de :
a) mise en contact de ladite première composition avec au moins un agent d'extraction organique pour former une seconde composition ;
b) distillation extractive de ladite seconde composition pour former :
   i) une troisième composition comprenant ledit agent d'extraction organique, 3,3,3-trifluoropropene (1243zf), trans-1,3,3,3-tetrafluoro-1-propene (1234ze-E) et optionnellement ou non au moins un composé sélectionné parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E); et
   ii) un courant comprenant le 2,3,3,3-tétrafluoro-1-propène,
c) récupération et séparation de ladite troisième composition pour un courant comprenant ledit agent d'extraction organique et un courant comprenant 3,3,3-trifluoropropene (1243zf), trans-1,3,3,3-tetrafluoro-1-propene (1234ze-E) et optionnellement ou non au moins un composé sélectionné parmi le groupe consistant en chlorométhane (40), 1,1-difluoroéthane (152a), chloropentafluoroéthane (115), 1,1,1,2-tetrafluoroethane (134a), et trans-1,2,3,3,3-pentafluoropropene (1225ye-E).

Ladite première composition peut comprendre entre 75 et 99,99 % en poids de 2,3,3,3-tétrafluoro-1-propène sur base du poids total de la première composition, avantageusement entre 80 et 99,9% en poids, de préférence entre 85 et 99,8% et en particulier entre 90 et 99,5% en poids de 2,3,3,3-tétrafluoro-1-propène sur base du poids total de la première composition.

Lorsqu'elle en contient, ladite première composition peut comprendre entre 0 et 2000 ppm poids de 1,1-difluoroéthane (152a) sur base du poids total de la première composition, avantageusement entre 0 et 1000 ppm poids, de préférence entre 0 et 500 ppm et en particulier entre 0 et 250 ppm en poids de 1,1-difluoroéthane (152a) sur base du poids total de la première composition.

Lorsqu'elle en contient, ladite première composition peut comprendre entre 0,01 et 25 % en poids de trans-1,3,3,3-tetrafluoro-1-propene (1234ze-E) sur base du poids total de la première composition, avantageusement entre 0.1 et 20% en poids, de préférence entre 0.2 et 15% en poids, en particulier entre 0.5 et 10% en poids, plus particulièrement entre 3 et 10% en poids de trans-1,3,3,3-tetrafluoro-1-propene (1234ze-E) sur base du poids total de la première composition.

Lorsqu'elle en contient, ladite première composition peut comprendre moins de 2000 ppm poids de 1,1,1,2-tetrafluoroethane (134a) sur base du poids total de la première composition, avantageusement entre 1 et 1000 ppm poids, de préférence entre 1 et 500 ppm et en particulier entre 1 et 250 ppm poids de 1,1,1,2-tetrafluoroethane (134a) sur base du poids total de la première composition.

Lorsqu'elle en contient, ladite première composition peut comprendre moins de 1 % poids de chlorométhane (40) sur base du poids total de la première composition, avantageusement entre 1 et 5000 ppm poids, de préférence entre 1 et 2000 ppm et en particulier entre 10 et 1500 ppm en poids de chlorométhane (40) sur base du poids total de la première composition.

Lorsqu'elle en contient, ladite première composition peut comprendre entre 0 et 2000 ppm poids de chloropentafluoroéthane (115) sur base du poids total de la première composition, avantageusement entre 1 et 1000 ppm poids, de préférence entre 1 et 500 ppm et en particulier entre 1 et 250 ppm en poids de chloropentafluoroéthane (115) sur base du poids total de la première composition.

Lorsqu'elle en contient, ladite première composition peut comprendre moins de 2000 ppm poids de cis-1,2,3,3,3-pentafluoropropene (1225ye) sur base du poids total de la première composition, avantageusement entre 1 et 1000 ppm poids, de préférence entre 1 et 500 ppm et en particulier entre 1 et 250 ppm poids de cis-1,2,3,3,3-pentafluoropropene (1225ye-Z) sur base du poids total de la première composition.

Lorsqu'elle en contient, ladite première composition peut comprendre moins de 1 % en poids de 1,1,1-trifluoropropene (1243zf) sur base du poids total de la première composition, avantageusement entre 0 et 5000 ppm poids, de préférence entre 1 et 2000 ppm et en particulier entre 1 et 1500 ppm poids de 1,1,1-trifluoropropene (1243zf) sur base du poids total de la première composition.

L'agent d'extraction de la présente invention est défini dans les revendications. Est également décrit un agent d'extraction qui est un solvant choisi parmi le groupe consistant en hydrocarbure, hydrohalocarbure, alcool, cétone, amine, ester, éther, aldéhyde, nitrile, carbonate, thioalkyle, amide et hétérocycle ; ou ledit agent d'extraction organique est difluoroéthylsilane, triéthylfluorosilane ou l'acide perfluorobutanoïque. De préférence, ledit agent d'extraction organique est un solvant choisi parmi le groupe consistant en cétone, ester, aldéhyde, éther, carbonate, et hétérocycle. De préférence, l'hétérocycle peut être un cycle carboné comprenant de 4 à 6 chaînons dont 1, 2 ou 3 chaînons sont des hétéroatomes sélectionnés parmi O ou N.

De préférence, les hydrocarbures sont sélectionnés parmi le groupe consistant en 2,2-dimethylbutane, 3-methylpentane, hexane, 2,4-dimethylpentane, cyclohexane, cyclohexene, methylcyclohexane, 2,4,4-trimethyl-1-pentene, 2,2,4-trimethyl-2-pentene, 1-methylcyclohexene, toluene, 2,3-dimethylhexane, octane, ethylcyclohexane, ethylbenzene, 1,4-dimethylbenzene, 1,3-dimethylbenzene, 1,2,3-trimethylcyclohexane, 1,2-dimethylbenzene, styrene.

De préférence, les hydrohalocarbures sont sélectionnés parmi le groupe consistant en Chloroethane, 3-chloro-1,1,1,2,2,3-hexafluoropropane, 2-chloro-1,1,1,3,3,3-hexafluoropropane, 1,1,1,3,3-pentafluoropropane, bromofluoromethane, 1-bromo-1,2-difluoroethylene, 1-chloro-1,1,2,3,3,3-hexafluoropropane, 1-chloro-1,1,2,2-tetrafluoropropane, 1-chloro-1,1,2,2,3,3-hexafluoropropane, 2-chloro-1,1,1,2-tetrafluoropropane, 1,1,1,2,3-pentafluoropropane, trichlorofluoromethane, 1-chloro-1,1-difluoropropane, 1,1,1-trifluoro-2-bromoethane, 1,1-dichloro-2,2,2-trifluoroethane, 1-chloro-2,2,3,3,3-pentafluoropropane, 1,2-dichloro-1,1,2-trifluoroethane, 1,1-dichloroethylene, 1,1,1,2,3,4,4,4-octafluorobutane, 2-chloro-2-fluoropropane, 1-chloro-2,2-difluoroethane, 2-chloropropane, bromoethane, dichloromethane, 2-chloro-1,1,1,3,3-pentafluoropropane, 1-chloro-1,2,2-trifluoropropane, iodomethane, 2,2-dichloro-1,1,1,3,3-pentafluoropropane, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, 1,1,2-trichloro-1,2,2-trifluoroethane, 1,2-dichloro-1,1,3,3,3-pentafluoropropane, 1-chloro-1,2,2,3-tetrafluoropropane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 1,3-dichloro-1,1,2,3,3-pentafluoropropane, 1,2-dichloro-3,3,3-trifluoropropene, 1,3-dichloro-1,1,2,2,3-pentafluoropropane, 1,1,1,2,2,3,4,5,5,5-decafluoropentane, 2,2-dichloro-1,1,1,3-tetrafluoropropane, 1-chloro-2,2-difluoropropane, 1,1-dichloroethane, 2-bromopropane, 1,2-dichloro-1,2,3,3,4,4-hexafluorocyclobutane, 1,1-dichloro-2,2-difluoroethane, 2,2-dichloro-1,1,3,3-tetrafluoropropane, trichloromethane, 2,3-dichloro-1,1,1,2-tetrafluoropropane, 1,1-dichloro-1,2,2,3-tetrafluoropropane, 1,3-dichloro-1,1,2,2-tetrafluoropropane, 1,1,1-trichloro-2,2-difluoroethane, 1,2-dichloro-1,2,3,3-tetrafluoropropane, chlorobromomethane, 2-chlorobutane, 1,3-dichloro-1,1,3,3-tetrafluoropropane, 1,3-dichloro-1,2,2,3-tetrafluoropropane, 1,2-dichloro-1,1,2,3-tetrafluoropropane, 3-bromopropene, 2,3-dichloro-1,1,1,3-tetrafluoropropane, 1,1-dichloro-2-fluoroethane, 1-bromopropane, 1,1-difluoro-1,2,2-trichloroethane, 1,1,2-trichloro-1,2-difluoroethane, iodoethane, 2-bromo-2-methylpropane, 1,2-dichloro-1-fluoroethane, 1,1,1-trichloroethane, 1-chloro-3-fluoropropane, 2,3-dichloro-1,1,1-trifluoropropane, tetrachloromethane, 1-chlorobutane, 1,3-dichloro-1,1,2-trifluoropropane, 1,1-dichloro-2,2,3-trifluoropropane, 1,3,3-trichloro-1,1,2,2-tetrafluoropropane, 2-chloro-2-methylbutane, 1,2-dichloroethane, 1,1,1-trichloro-2,2,3,3-tetrafluoropropane, 1,1,3-trichloro-1,2,2,3-tetrafluoropropane, 1,3-dichloro-1,2,2-trifluoropropane, trichloroethene, 1,1-dichloropropane, 1,2-dichloro-2-fluoropropane, 2-iodopropane, dichlorobromomethane, 2-bromobutane, 2,2-difluorotetrachloroethane, 1,1,2,2-tetrachloro-1,2-difluoro-ethane, 1-fluorohexane, 1,3-dichloro-1,2,3-trifluoropropane, 2,3-dichloro-1-propene, 1,2-dichloropropane, 3-chloropentane, trichloroacetaldehyde, isoamylchloride, 1,1,1-trichloro-2,2,3-trifluoropropane, 1-chloro-4-fluorobutane, 1-bromo-3-fluoropropane, 1-bromobutane, 1,1,2-trichloro-2-fluoroethane, 1-iodopropane, 1,1,3-trichloro-1,2,2-trifluoropropane, 1,1,3-trichloro-2,2,3-trifluoropropane, cis-1,3-dichloropropene, 2,2-dichlorobutane, bromotrichloromethane, 1,1,1,2-tetrachloro-2-fluoroethane, 1-bromo-2-chloroethane, 2-bromo-2-methylbutane, trans-1,3-dichloropropene, 2-fluorotoluene, 1,1,2-trichloroethane, 1,1,1-trichloro-3-fluoropropane, 3,3,3-trichloro-1-propene, 1-chloro-3,3-dimethylbutane, 1,1,1,2-tetrachloro-3,3,3-trifluoropropane, 2-bromopentane, trichloroacetylchloride, 2,3-dichlorobutane, 1,1,3,3-tetrachloro-1,2,2-trifluoropropane, 1,1,1,3-tetrachloro-2,2,3-trifluoropropane, 1-bromo-3-methylbutane, 1,3-dichloro-trans-2-butene, 1,3-dichloropropane, tetrachloroethene, 1,2-dibromo-1-fluoroethane, 1,2,2-trichloropropane, 1,2-dichlorobutane, 1,2,2,3-tetrachloro-3,3-difluoropropane, 2,3-dichloro-2-methylbutane, 1-bromopentane, 1,2-dichloro-2-butene, 1-iodobutane, 1,2-dibromoethane, chlorobenzene, 1,1,2-trichloropropane, 1,3-dichlorobutane, pentachlorofluoroethane, 1,2-dibromopropane, 1,2,3-trichloropropene, 1-chloro-3-bromopropane, 1,1,3,3-tetrachloro-1-fluoropropane, 1,1,2,2,3-pentchloro-3,3-difluoropropane, 1,1,2,2-tetrachloroethane, 1,2-dichloropentane, tribromomethane.

De préférence, les alcools sont sélectionnés parmi le groupe consistant en methanol, 2,2,2-trifluoroethanol, 1,1,1-trifluoro-2-propanol, ethanol, pentafluoro-1-propanol, 2-propanol, tert-butanol, 2,2-difluoroethanol, propanol, 2-allyloxyethanol, 2-butanol, 2-methyl-2-butanol, isobutanol, 2,2,3,3-tetraflouro-1-propanol, 2,2-dimethyl-1-propanol, 3-pentanol, 1-butanol, 1-methoxy2-propanol, 1-(dimethylamino)-2-propanol, 3-methyl-3-pentanol, 1-chloro-2-methyl-2-propanol, 4,4,4-trifluorobutanol, 3-fluoropropanol, 2-chloroethanol, 2-methoxyl-propanol, 1-ethoxy-2-propanol, 4-methyl-2-pentanol, 1,2-octanediol, 2-chloro-1-propanol, 2-(dimethylamino)-ethanol, 3-hexanol, 2-hexanol, 2-ethoxy-1-propanol, 1-pentanol, 3,3,4,4,5,5,6,6-octafluoro-1-pentanol, 2,3-dimethylbutanol, 2-ethyl-1-butanol, 2-methyl-1-pentanol, 2-propoxyethanol, 1-propoxy-2-propanol, 2-aminophenol.

De préférence, les cétones sont sélectionnées parmi le groupe consistant en 1,1,1-trifluoro-2-propanone, propanone, butanone, 3-pentanone, 2-pentanone, 3,3-dimethyl-2-butanone, 4-methyl-2-pentanone, 2-hexanone, 5-hexen-2-one, 4-methyl-2-hexanone.

De préférence, les amines sont sélectionnées parmi le groupe consistant en Ethylamine, isopropylamine, ethylmethylamine, 2-amino-2-methylpropane, n-propylamine, isopropylmethylamine, diethylamine, 2-butanamine, n-methylpropylamine, 1-butylamine, diisopropylamine, 3-methyl-2-butanamine, 3-pentylamine, n-methylbutylamine, 1-methoxy-2-propanamine, 2-methoxyethanamine, 2-methoxy-1propanamine, n-pentylamine, n-methylhydroxylamine, dipropylamine, 2-ethoxyethanamine, n-methyl-1,2-ethanediamine, pyridine, 1,2-diaminoethane, 1,2-propanediamine, 2-ethylbutylamine, n-ethylethylenediamine, 1,1-diethoxy-n,n-dimethylmethanamine, 2-methylpyridine, 4-methyl-2-hexanamine, hexylamine, cyclohexylamine, n-ethyl-2-dimethylaminoethylamine, 1,3-propanediamine, 2-heptanamine, n,n-diethylethylenediamine, 2,6-dimethylpyridine, 4-methylpyridine, n,n'-diethyl-1,2-ethanediamine, dimethylethanolamine.

De préférence, les esters sont sélectionnés parmi le groupe consistant en Methylformate, methylacetate, isopropylformate, ethylacetate, n-propylformate, isopropylacetate, tert-butylacetate, ethylpropionate, sec-butylacetate, diethylcarbonate, n-butylacetate, bromoaceticacidmethylester, methylhexanoate.

De préférence, les éthers sont sélectionnés parmi le groupe consistant en 2,2,2-trifluoroethylmethylether, 1,1,2,2-tetrafluoroethylmethylether, 2-methoxy-1-propene, diethylether, ethoxy-ethene, dimethoxymethane, methylcyclopropylether, 2-ethoxy-propane, methyl-t-butylether, ethyl1,1,2,2-tetrafluoroethylether, chloromethoxymethane, diisopropylether, 2-ethoxy-2-methyl-propane, 2-ethoxy-butane, 1-methoxy-2-methyl-butane, 2,2-dimethoxypropane, 1-ethoxy-2-methylpropane, 1,2-dimethoxyethane, diethoxymethane, di-n-propylether, 1-ethoxybutane, 1-methoxy-pentane, 1,2-dimethoxypropane, isopropyl-isobutyl-ether, 1,1-diethoxyethane, trimethoxymethane, 1,1-dichloro-2,2-difluoroethylmethylether, 2,2-diethoxypropane, isobutyl-tert-butylether, sec-butyl-tert-butylether, 1,1-diethoxypropane, 2-methoxyethanol, 2-chloro-1,1-dimethoxyethane, methoxycyclohexane, ethoxyethanol, di-n-butylether, 1-ethoxy-hexane, 1-methoxy-2-acetoxypropane, 1,1,1-triethoxyethane.

De préférence, les aldéhydes sont sélectionnés parmi le groupe consistant en Acetaldehyde, ethanedial, isobutanal, methylglyoxal, 2-methylbutanal, 2,6-dimethyl-5-heptenal, hexanal.

De préférence, les nitriles sont sélectionnés parmi le groupe consistant en acétonitrile, propionitrile, butyronitrile, valeronitrile, (methyleneamino)acetonitrile.

De préférence, le carbonate est le diethylcarbonate.

De préférence, l'amide est l'éthanethioamide.

De préférence, les thioalkyles sont sélectionnés parmi le groupe consistant en éthanethiol, dimethylsulfide, 2-propanetiol, 4-methoxy-2-methyl-2-butanethiol, tert-butylthiol, 1-propanethiol, thiophene, 2-butanethiol, 2-methyl-1-propanethiol, diethylsulfide, butanethiol, 3, mercapto-1,2-propanediol, tetrahydrothiophene, 1-pentanethiol.

De préférence, les hétérocycles sont sélectionnés parmi le groupe consistant en furane, 1,2-epoxypropane, tetrahydrofurane, dioxane, 1,3-dioxane, piperidine, 1,3,5-trioxane, n-methylmorpholine, 2-methylpyrazine, 1-methylpiperazine, n-ethyl-morpholine, 2,6-dimethylmorpholine, 3-furfural.

Ledit agent d'extraction organique également décrit peut être Chloroethane, 3-chloro-1,1,1,2,2,3-hexafluoropropane, 2-chloro-1,1,1,3,3,3-hexafluoropropane, 1,1,1,3,3-pentafluoropropane, ethylamine, bromofluoromethane, 1-bromo-1,2-difluoroethylene1-chloro-1,1,2,3,3,3-hexafluoropropane, 1-chloro-1,1,2,2-tetrafluoropropane, acetaldehyde, 1-chloro-1,1,2,2,3,3-hexafluoropropane, 1,1,1-trifluoro-2-propanone, 2-chloro-1,1,1,2-tetrafluoropropane, 1,1,1,2,3-pentafluoropropane, trichlorofluoromethane, 1-chloro-1,1-difluoropropane, 1,1,1-trifluoro-2-bromoethane, 1,1-dichloro-2,2,2-trifluoroethane, 1-chloro-2,2,3,3,3-pentafluoropropane, 1,2-dichloro-1,1,2-trifluoroethane, 2,2,2-trifluoroethylmethylether, 1,1-dichloroethylene, furane, methylformate, isopropylamine, 1,1,2,2-tetrafluoroethylmethylether, 1,1,1,2,3,4,4,4-octafluorobutane, 2-methoxy-1-propene, diethylether, 1,2-epoxypropane, ethanethiol, 2-chloro-2-fluoropropane, ethoxy-ethene, 1-chloro-2,2-difluoroethane, ethylmethylamine, dimethylsulfide, 2-chloropropane, bromoethane, dichloromethane, dimethoxymethane, 2-chloro-1,1,1,3,3-pentafluoropropane, 1-chloro-1,2,2-trifluoropropane, iodomethane, 2,2-dichloro-1,1,1,3,3-pentafluoropropane, 2-amino-2-methylpropane, methylcyclopropylether, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, n-propylamine, 1,1,2-trichloro-1,2,2-trifluoroethane, 2,2-dimethylbutane, isopropylmethylamine, ethanedial, 1,2-dichloro-1,1,3,3,3-pentafluoropropane, 1-chloro-1,2,2,3-tetrafluoropropane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 2-propanethiol, 1,3-dichloro-1,1,2,3,3-pentafluoropropane, 1,2-dichloro-3,3,3-trifluoropropene, 1,3-dichloro-1,1,2,2,3-pentafluoropropane, 1,1,1,2,2,3,4,5,5,5-decafluoropentane, 2-ethoxy-propane, 2,2-dichloro-1,1,1,3-tetrafluoropropane, 1-chloro-2,2-difluoropropane, methyl-t-butylether, diethylamine, propanone, methylacetate, 1,1-dichloroethane, ethyl1,1,2,2-tetrafluoroethylether, 4-methoxy-2-methyl-2, butanethiol, 2-bromopropane, chloromethoxymethane, 1,2-dichloro-1,2,3,3,4,4-hexafluorocyclobutane, 1,1-dichloro-2,2-difluoroethane, 2,2-dichloro-1,1,3,3-tetrafluoropropane, difluorodiethylsilane, 2-butanamine, 2,3-dichloro-1,1,1,2-tetrafluoropropane, n-methylpropylamine, 3-methylpentane, 1,1-dichloro-1,2,2,3-tetrafluoropropane, tert-butylthiol, isobutanal, methanol, tetrahydrofurane, 1,3-dichloro-1,1,2,2-tetrafluoropropane, 1,1,1-trichloro-2,2-difluoroethane, 1,2-dichloro-1,2,3,3-tetrafluoropropane, 1-propanethiol, chlorobromomethane, 2-chlorobutane, isopropylformate, diisopropylether, 1,3-dichloro-1,1,3,3-tetrafluoropropane, hexane, 1,3-dichloro-1,2,2,3-tetrafluoropropane, 1,2-dichloro-1,1,2,3-tetrafluoropropane, 3-bromopropene, 2,3-dichloro-1,1,1,3-tetrafluoropropane, 1,1-dichloro-2-fluoroethane, 1-bromopropane, 1,1-difluoro-1,2,2-trichloroethane, 1,1,2-trichloro-1,2-difluoroethane, methylglyoxal, iodoethane, 2-ethoxy-2-methyl-propane, 2-bromo-2-methylpropane, 1,2-dichloro-1-fluoroethane, 1,1,1-trichloroethane, 2,2,2-trifluoroethanol, 1-chloro-3-fluoropropane, 1,1,1-trifluoro-2-propanol, 2,3-dichloro-1,1,1-trifluoropropane, ethylacetate, 1-butylamine, 1-chlorobutane, ethanol, butanone, 2,4-dimethylpentane, cyclohexane, n-propylformate, 2-ethoxy-butane, acetonitrile, pentafluoro-1-propanol, 2-propanol, tert-butanol, 1,3-dichloro-1,1,2-trifluoropropane, 1-methoxy-2-methyl-butane, 1,1-dichloro-2,2,3-trifluoropropane, cyclohexene, 2,2-dimethoxypropane, 1,3,3-trichloro-1,1,2,2-tetrafluoropropane, 2-chloro-2-methylbutane, 1,2-dichloroethane, 1-ethoxy-2-methylpropane, diisopropylamine, thiophene, 2-butanethiol, 1,1,1-trichloro-2,2,3,3-tetrafluoropropane, 1,2-dimethoxyethane, 3-methyl-2-butanamine, 1,1,3-trichloro-1,2,2,3-tetrafluoropropane, 1,3-dichloro-1,2,2-trifluoropropane, diethoxymethane, 1,1-dichloropropane, 2-methyl-1-propanethiol, 1,2-dichloro-2-fluoropropane, isopropylacetate, 2-iodopropane, di-n-propylether, 3-pentylamine, n-methylbutylamine, 2-bromobutane, 2,2-difluorotetrachloroethane, diethylsulfide, 1-ethoxybutane, 1,1,2,2-tetrachloro-1,2-difluoro-ethane, 1-fluorohexane, 1-methoxy-2-propanamine, 1,3-dichloro-1,2,3-trifluoropropane, 2,3-dichloro-1-propene, 2-methoxyethanamine, 2,2-difluoroethanol, 2-methylbutanal, 1,2-dichloropropane, propanol, tert-butylacetate, propionitrile, 3-chloropentane, trichloroacetaldehyde, 2-allyloxyethanol, butanethiol, isoamylchloride, 1-methoxy-pentane, ethylpropionate, 2-butanol, 1,2-dimethoxypropane, isopropyl-isobutyl-ether, 1,1,1-trichloro-2,2,3-trifluoropropane, methylcyclohexane, 1-chloro-4-fluorobutane, 1-bromo-3-fluoropropane, 2,4,4-trimethyl-1-pentene, dioxane, 1-bromobutane, 3-pentanone, 1,1,2-trichloro-2-fluoroethane, 1,1-diethoxyethane, 2-pentanone, 2-methyl-2-butanol, 1-iodopropane, 2-methoxy-1propanamine, 1,1,3-trichloro-1,2,2-trifluoropropane, 1,1,3-trichloro-2,2,3-trifluoropropane, trimethoxymethane, cis-1,3-dichloropropene, 2,2-dichlorobutane, n-pentylamine, 1,1-dichloro-2,2-difluoroethylmethylether, 2,2,4-trimethyl-2-pentene, 1,1,1,2-tetrachloro-2-fluoroethane, 1,3-dioxane, 3,3-dimethyl-2, butanone, piperidine, 1-bromo-2-chloroethane, isobutanol, 2-bromo-2-methylbutane, dipropylamine, 2,2,3,3-tetraflouro-1-propanol, 2-ethoxyethanamine, triethylfluorosilane, 1-methylcyclohexene, toluene, trans-1,3-dichloropropene, sec-butylacetate, 2-fluorotoluene, 2,2-dimethyl-1-propanol, 1,1,1-trichloro-3-fluoropropane, n-methyl-1,2-ethanediamine, 2,2-diethoxypropane, 1,3,5-trioxane, 3,3,3-trichloro-1-propene, 1-chloro-3,3-dimethylbutane, pyridine, 2,3-dimethylhexane, 1,1,1,2-tetrachloro-3,3,3-trifluoropropane, n-methylmorpholine, 3-pentanol, 4-methyl-2-pentanone, 1,2-diaminoethane, isobutyl-tert-butylether, 2-bromopentane, butyronitrile, 1-butanol, 2,3-dichlorobutane, sec-butyl-tert-butylether, 1-methoxy2-propanol, 1,1,3,3-tetrachloro, 1,2,2-trifluoropropane, 1,2-propanediamine, 2,6-dimethyl-5-heptenal, perfluorobutanoicacid, 1,1,1,3-tetrachloro-2,2,3-trifluoropropane, 1-bromo-3-methylbutane, 1,3-dichloro-trans-2-butene, 1,3-dichloropropane,1-(dimethylamino)-2-propanol, tetrahydrothiophene, 3-methyl-3-pentanol, 1,2-dibromo-1-fluoroethane, 1,1-diethoxypropane, 1,2,2-trichloropropane, 1-chloro-2-methyl-2, propanol, 2-methoxyethanol, 1,2-dichlorobutane, 4,4,4-trifluorobutanol, 2-ethylbutylamine, octane, diethylcarbonate, n-butylacetate, 1-pentanethiol, 1,2,2,3-tetrachloro-3,3-difluoropropane, 2-chloro-1,1-dimethoxyethane, 2-hexanone, n-ethylethylenediamine, 3-fluoropropanol, 5-hexen-2-one, 2,3-dichloro-2-methylbutane, 1,1-diethoxy-n,n-dimethylmethanamine, 2-methylpyridine, 1-bromopentane, 2-methoxy1-propanol, 1,2-dichloro-2-butene, 1-iodobutane, 1-ethoxy-2-propanol, hexanal, 4-methyl-2-pentanol, 1,2-dibromoethane, chlorobenzene, ethylcyclohexane, bromoaceticacidmethylester, 1,1,2-trichloropropane, 1,2-octanediol, 4-methyl-2-hexanamine, hexylamine, 2-chloro-1-propanol, methoxycyclohexane, 2-(dimethylamino)-ethanol, 1,3-dichlorobutane, cyclohexylamine, n-ethyl-2-dimethylaminoethylamine, ethoxyethanol, 3-hexanol, 2-hexanol, ethylbenzene, 2-methylpyrazine, 2-ethoxy-1-propanol, 1-pentanol, 1-methylpiperazine, n-ethyl-morpholine, 1,4-dimethylbenzene, 1,3-dimethylbenzene, 1,3-propanediamine, di-n-butylether, 3,3,4,4,5,5,6,6-octafluoro-1-pentanol, valeronitrile, (methyleneamino)acetonitrile, 1,2-dibromopropane, 2-heptanamine, 1,2,3-trimethylcyclohexane, 2,3-dimethylbutanol, 1-ethoxy-hexane, 1-chloro-3-bromopropane, 3-furfural, n,n-diethylethylenediamine, 2,6-dimethylpyridine, 1,1,3,3-tetrachloro-1-fluoropropane, 4-methyl-2-hexanone, 1,2-dimethylbenzene, 1,1,2,2,3-pentchloro-3,3-difluoropropane, 1-methoxy-2-acetoxypropane, 1,1,1-triethoxyethane, styrene, 4-methylpyridine, n,n'-diethyl-1,2-ethanediamine, 2,6-dimethylmorpholine, 2-ethyl-1-butanol, 1,2-dichloropentane, 2-methyl-1-pentanol, methylhexanoate, 2-propoxyethanol, dimethylethanolamine, 1-propoxy-2-propanol.

Avantageusement, ledit agent d'extraction organique également décrit peut être Chloroethane, 1,1,1,3,3-pentafluoropropane, bromofluoromethane, 1-bromo-1,2-difluoroethylene, 1-chloro-1,1,2,2-tetrafluoropropane, acetaldehyde, 1,1,1-trifluoro-2-propanone, 2-chloro-1,1,1,2-tetrafluoropropane, 1,1,1,2,3-pentafluoropropane, 1-chloro-1,1-difluoropropane, 1,1,1-trifluoro-2-bromoethane, 1,1-dichloro-2,2,2-trifluoroethane, 1-chloro-2,2,3,3,3-pentafluoropropane, 1,2-dichloro-1,1,2-trifluoroethane, 2,2,2-trifluoroethylmethylether, furane, methylformate, isopropylamine, 1,1,2,2-tetrafluoroethylmethylether, 2-methoxy-1-propene, diethylether, 1,2-epoxypropane, ethanethiol, 2-chloro-2-fluoropropane, ethoxy-ethene, 1-chloro-2,2-difluoroethane, ethylmethylamine, dimethylsulfide, 2-chloropropane, bromoethane, dimethoxymethane, 2-chloro-1,1,1,3,3-pentafluoropropane, 1-chloro-1,2,2-trifluoropropane, 2,2-dichloro-1,1,1,3,3-pentafluoropropane, 2-amino-2-methylpropane, methylcyclopropylether, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, 1,1,2-trichloro-1,2,2-trifluoroethane, isopropylmethylamine, ethanedial, 1,2-dichloro-1,1,3,3,3-pentafluoropropane, 1-chloro-1,2,2,3-tetrafluoropropane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 2-propanethiol, 1,2-dichloro-3,3,3-trifluoropropene, 2-ethoxy-propane, 2,2-dichloro-1,1,1,3-tetrafluoropropane, 1-chloro-2,2-difluoropropane, methyl-t-butylether, diethylamine, propanone, methylacetate, ethyl1,1,2,2-tetrafluoroethylether, 4-methoxy-2-methyl-2-butanethiol, 2-bromopropane, 1,1-dichloro-2,2-difluoroethane, 2,2-dichloro-1,1,3,3-tetrafluoropropane, difluorodiethylsilane, 2,3-dichloro-1,1,1,2-tetrafluoropropane, 1,1-dichloro-1,2,2,3-tetrafluoropropane, tert-butylthiol, isobutanal, tetrahydrofurane, 1,3-dichloro-1,1,2,2-tetrafluoropropane, 1,1,1-trichloro-2,2-difluoroethane, 1,2-dichloro-1,2,3,3-tetrafluoropropane, 1-propanethiol, 2-chlorobutane, isopropylformate, diisopropylether, 1,3-dichloro-1,2,2,3-tetrafluoropropane, 1,2-dichloro-1,1,2,3-tetrafluoropropane, 2,3-dichloro-1,1,1,3-tetrafluoropropane, 1-bromopropane, 1,1-difluoro-1,2,2-trichloroethane, 1,1,2-trichloro-1,2-difluoroethane, methylglyoxal, 2-ethoxy-2-methyl-propane, 2-bromo-2-methylpropane, 1-chloro-3-fluoropropane, 1,1,1-trifluoro-2-propanol, 2,3-dichloro-1,1,1-trifluoropropane, ethylacetate, 1-chlorobutane, butanone, n-propylformate, 2-ethoxy-butane, pentafluoro-1-propanol, tert-butanol, 1,3-dichloro-1,1,2-trifluoropropane, 1-methoxy-2-methyl-butane, 1,1-dichloro-2,2,3-trifluoropropane, cyclohexene, 2,2-dimethoxypropane, 1,3,3-trichloro-1,1,2,2-tetrafluoropropane, 2-chloro-2-methylbutane, 1-ethoxy-2-methylpropane, diisopropylamine, 2-butanethiol, 1,1,1-trichloro-2,2,3,3-tetrafluoropropane, 1,2-dimethoxyethane, 1,1,3-trichloro-1,2,2,3-tetrafluoropropane, 1,3-dichloro-1,2,2-trifluoropropane, diethoxymethane, 2-methyl-1-propanethiol, isopropylacetate, 2-iodopropane, di-n-propylether, 2-bromobutane, diethylsulfide, 1-ethoxybutane, 1-fluorohexane, 1-methoxy-2-propanamine, 1,3-dichloro-1,2,3-trifluoropropane, 2-methoxyethanamine, 2-methylbutanal, tert-butylacetate, propionitrile, 3-chloropentane, 2-allyloxyethanol, butanethiol, isoamylchloride, 1-methoxy-pentane, ethylpropionate, 2-butanol, 1,2-dimethoxypropane, isopropyl-isobutyl-ether, 1,1,1-trichloro-2,2,3-trifluoropropane, 1-chloro-4-fluorobutane, 1-bromo-3-fluoropropane, 2,4,4-trimethyl-1-pentene, dioxane, 1-bromobutane, 3-pentanone, 1,1-diethoxyethane, 2-pentanone, 2-methyl-2-butanol, 2-methoxy-1propanamine, 1,1,3-trichloro-1,2,2-trifluoropropane, 1,1,3-trichloro-2,2,3-trifluoropropane, trimethoxymethane, 1,1-dichloro-2,2-difluoroethylmethylether, 2,2,4-trimethyl-2-pentene, 1,3-dioxane, 3,3-dimethyl-2-butanone, piperidine, 2-bromo-2-methylbutane, dipropylamine, 2,2,3,3-tetraflouro-1-propanol, 2-ethoxyethanamine, triethylfluorosilane, 1-methylcyclohexene, toluene, sec-butylacetate, 2-fluorotoluene, 2,2-dimethyl-1-propanol, n-methyl-1,2-ethanediamine, 2,2-diethoxypropane, 1,3,5-trioxane, 1-chloro-3,3-dimethylbutane, pyridine, 1,1,1,2-tetrachloro-3,3,3-trifluoropropane, n-methylmorpholine, 3-pentanol, 4-methyl-2-pentanone, 1,2-diaminoethane, isobutyl-tert-butylether, 2-bromopentane, butyronitrile, 1-butanol, sec-butyl-tert-butylether, 1-methoxy2-propanol, 1,1,3,3-tetrachloro-1,2,2-trifluoropropane, 1,2-propanediamine, 2,6-dimethyl-5-heptenal, 1,1,1,3-tetrachloro-2,2,3-trifluoropropane, 1-bromo-3-methylbutane, 1-(dimethylamino)-2-propanol, tetrahydrothiophene, 3-methyl-3-pentanol, 1,1-diethoxypropane, 2-methoxyethanol, 4,4,4-trifluorobutanol, diethylcarbonate, n-butylacetate, 1-pentanethiol, 2-chloro-1,1-dimethoxyethane, 2-hexanone, n-ethylethylenediamine, 5-hexen-2-one, 1,1-diethoxy-n,n-dimethylmethanamine, 2-methylpyridine, 1-bromopentane, 2-methoxy1-propanol, 1-ethoxy-2-propanol, hexanal, 4-methyl-2-pentanol, 1,2-octanediol, methoxycyclohexane, 2-(dimethylamino)-ethanol, cyclohexylamine, n-ethyl-2-dimethylaminoethylamine, ethoxyethanol, 3-hexanol, 2-hexanol, ethylbenzene, 2-methylpyrazine, 2-ethoxy-1-propanol, 1-pentanol, 1-methylpiperazine, n-ethyl-morpholine, 1,4-dimethylbenzene, 1,3-dimethylbenzene, 1,3-propanediamine, di-n-butylether, 3,3,4,4,5,5,6,6-octafluoro-1-pentanol, valeronitrile, (methyleneamino)acetonitrile, 2,3-dimethylbutanol, 1-ethoxy-hexane, 3-furfural, 2,6-dimethylpyridine, 4-methyl-2-hexanone, 1,2-dimethylbenzene, 1-methoxy-2-acetoxypropane, 1,1,1-triethoxyethane, styrene, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, dimethylethanolamine, 1-propoxy-2-propanol.

De préférence, ledit agent d'extraction organique également décrit peut être Acetaldehyde, methylformate, 2-methoxy-1-propene, 1,2-epoxypropane, ethoxy-ethene, dimethoxymethane, 1-chloro-1,2,2-trifluoropropane, 3-chloro-1,1,1-trifluoropropane, ethanedial, 2-chloro-1,1,1,3-tetrafluoropropane, propanone, methylacetate, 4-methoxy-2-methyl-2-butanethiol, difluorodiethylsilane, isobutanal, isopropylformate, methylglyoxal, 2,3-dichloro-1,1,1-trifluoropropane, ethylacetate, butanone, n-propylformate, 1,3-dichloro-1,1,2-trifluoropropane, 1,1-dichloro-2,2,3-trifluoropropane, 1,2-dimethoxyethane, 1,3-dichloro-1,2,2-trifluoropropane, isopropylacetate, diethylsulfide, 1,3-dichloro-1,2,3-trifluoropropane, 2-methylbutanal, 2-allyloxyethanol, ethylpropionate, 1,2-dimethoxypropane, dioxane, 3-pentanone, 2-pentanone, trimethoxymethane, 1,3-dioxane, 3,3-dimethyl-2-butanone, pyridine, n-methylmorpholine, 4-methyl-2-pentanone, butyronitrile, 1-methoxy2-propanol, diethylcarbonate, n-butylacetate, 2-hexanone, 5-hexen-2-one, 2-methoxy1-propanol, 1-ethoxy-2-propanol, hexanal, 2-(dimethylamino)-ethanol, 2-methylpyrazine, 2-ethoxy-1-propanol, 1-methylpiperazine, n-ethyl-morpholine, valeronitrile, 4-methyl-2-hexanone, 1-methoxy-2-acetoxypropane, 2,6-dimethylmorpholine, methylhexanoate, 2-propoxyethanol, 1-propoxy-2-propanol.

En particulier, ledit agent d'extraction organique également décrit peut être 2-methoxy-1-propene, 1,2-epoxypropane, ethoxy-ethene, dimethoxymethane, methylacetate, isobutanal, isopropylformate, ethylacetate, butanone, n-propylformate, 1,2-dimethoxyethane, isopropylacetate, 2-methylbutanal, ethylpropionate, 1,2-dimethoxypropane, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 2-hexanone, 5-hexen-2-one, 1-ethoxy-2-propanol, hexanal, 2-(dimethylamino)-ethanol, 2-methylpyrazine, 1-methylpiperazine, valeronitrile, 4-methyl-2-hexanone, 1-methoxy-2-acetoxypropane, 2,6-dimethylmorpholine, methylhexanoate, 1-propoxy-2-propanol.

L'agent d'extraction organique à utiliser peut être sélectionné en fonction des composés présents dans ladite première composition. Ainsi, l'agent d'extraction organique peut être sélectionné en fonction du facteur de séparation et de la capacité d'absorption établi pour une composition particulière. Outre ces deux critères, le choix de l'agent d'extraction organique peut se baser optionnellement sur d'autres critères commerciaux ou environnementaux, tels que par exemple le coût de l'agent d'extraction organique, sa disponibilité sur le marché, ses propriétés toxiques ou inflammable. De plus, selon un mode de réalisation particulier, afin d'optimiser le fonctionnement des colonnes de distillation utilisées à l'étape b) et c) du présent procédé de purification du 2,3,3,3-tetrafluoro-1-propene, le point d'ébullition de l'agent d'extraction organique peut être de 10°C à 200°C, avantageusement de 10°C à 190°C, de préférence de 10°C à 180°C, en particulier de 10°C à 170°C, plus particulièrement de 10°C à 160°C, et de manière privilégiée de 10°C à 150°C.

Selon un mode de réalisation préféré, ledit agent d'extraction organique a un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle
γ_{1,S} représente le coefficient d'activité du 2,3,3,3-tétrafluoro-1-propène dans ledit agent d'extraction organique à dilution infinie,
P1 représente la pression de vapeur saturante du 2,3,3,3-tétrafluoro-1-propène,
γ_{2,S} représente le coefficient d'activité d'un des composés consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf), dans ledit agent d'extraction organique à dilution infinie ; avantageusement le coefficient d'activité du 3,3,3-trifluoropropene (1243zf) ou du trans-1,3,3,3-tetrafluoro-1-propene (1234ze-E),
P2 représente la pression de vapeur saturante d'un des composés consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) ; avantageusement la pression de vapeur saturante du 3,3,3-trifluoropropene (1243zf) ou du trans-1,3,3,3-tetrafluoro-1-propene (1234ze-E).

Avantageusement, le facteur de séparation S_{1,2} est supérieur ou égal à 1,2, de préférence supérieur ou égal à 1,4, plus préférentiellement supérieur ou égal à 1,6, en particulier supérieur ou égal à 1,8, plus particulièrement, supérieur ou égal à 2,0.

Selon un mode de réalisation préféré, ledit agent d'extraction organique a une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité d'un des composés consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) dans ledit agent d'extraction organique à dilution infinie ; avantageusement le coefficient d'activité de 3,3,3-trifluoropropene (1243zf) ou de trans-1,3,3,3-tetrafluoro-1-propene (1234ze-E).

Avantageusement, la capacité d'absorption C_{2,S} est supérieure ou égale à 0,40, de préférence supérieure ou égale à 0,60, plus préférentiellement supérieure ou égale à 0,80, en particulier supérieure ou égale à 1,0.

Selon un mode de réalisation préféré, ledit agent d'extraction organique peut avoir un facteur de séparation S_{1,2} supérieur ou égal à 1,1, avantageusement supérieur ou égal à 1,2, de préférence supérieur ou égal à 1,4, plus préférentiellement supérieur ou égal à 1,6, en particulier supérieur ou égal à 1,8 ; et une capacité d'absorption C_{2,S} supérieure ou égale à 0,20, avantageusement supérieure ou égale à 0,40, de préférence supérieure ou égale à 0,60, plus préférentiellement supérieure ou égale à 0,80, en particulier supérieure ou égal à 1,0.

Ladite première composition peut être une composition azéotropique ou quasi-azéotropique comprenant 2,3,3,3-tetrafluoro-1-propène et d'au moins un des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf). Avantageusement, ladite première composition peut être une composition azéotropique ou quasi-azéotropique comprenant 2,3,3,3-tetrafluoro-1-propène et au moins deux, au moins trois, au moins quatre, au moins cinq, au moins six ou l'ensemble des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf). De préférence, ladite première composition peut être une composition azéotropique ou quasi-azéotropique comprenant 2,3,3,3-tetrafluoro-1-propène, 3,3,3-trifluoropropene (1243zf), trans-1,3,3,3-tetrafluoro-1-propene (1234ze-E) et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E).

En fonction du ou des composés à éliminer dans ladite première composition, ledit facteur de séparation et ladite capacité d'absorption pourront être calculés pour un couple binaire particulier consistant en 2,3,3,3-tetrafluoro-1-propène et un des composés choisi parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf). Ainsi, pour sélectionner ledit agent d'extraction organique apte à être utilisé dans l'étape b) de distillation extractive, le facteur de séparation et la capacité d'absorption peuvent être calculés par exemple pour un couple binaire 2,3,3,3-tetrafluoro-1-propène et 3,3,3-trifluoropropene (1243zf) si ladite première composition comprend ces deux composés. Le facteur de séparation S_{1,2} permet de déterminer la capacité d'un agent d'extraction organique à séparer deux ou plusieurs composés. La capacité d'absorption C_{2,S} permet de déterminer la quantité de solvant à utiliser pour obtenir la séparation entre les composés considérés. En outre, si l'on souhaite optimiser la séparation d'une première composition, le facteur de séparation et la capacité d'absorption peuvent être calculés pour plusieurs couples binaires, et l'agent d'extraction organique est ainsi sélectionné sur base des valeurs obtenues pour tout ou partie des couples binaires considérés.

En particulier, ladite première composition peut donc être une composition azéotropique ou quasi-azéotropique comprenant 2,3,3,3-tetrafluoro-1-propène, 3,3,3-trifluoropropene (1243zf) et trans-1,3,3,3-tetrafluoro-1-propene (1234ze-E). La teneur en chacun des composés dans cette composition particulière est telle qu'exprimée ci-dessus en référence aux teneurs individuelles de chacun des composés. Ainsi, la seconde composition peut comprendre 2,3,3,3-tetrafluoro-1-propène, 3,3,3-trifluoropropene (1243zf), trans-1,3,3,3-tetrafluoro-1-propene (1234ze-E) et ledit agent d'extraction organique.

Ledit agent d'extraction organique peut avoir un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle γ_{1,S} représente le coefficient d'activité du 2,3,3,3-tétrafluoro-1-propène dans ledit agent d'extraction organique à dilution infinie, P1 représente la pression de vapeur saturante du 2,3,3,3-tétrafluoro-1-propène, γ_{2,S} représente le coefficient d'activité du 3,3,3-trifluoropropene (1243zf) dans ledit agent d'extraction organique à dilution infinie et P2 représente la pression de vapeur saturante du 3,3,3-trifluoropropene (1243zf), avantageusement, ledit agent d'extraction organique peut avoir un facteur de séparation supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0 ; et ledit agent d'extraction organique peut avoir un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle γ_{1,S} représente le coefficient d'activité du 2,3,3,3-tétrafluoro-1-propène dans ledit agent d'extraction organique à dilution infinie, P1 représente la pression de vapeur saturante du 2,3,3,3-tétrafluoro-1-propène, γ_{2,S} représente le coefficient d'activité du trans-1,3,3,3-tetrafluoro-1-propene (1234ze-E) dans ledit agent d'extraction organique à dilution infinie et P2 représente la pression de vapeur saturante du trans-1,3,3,3-tetrafluoro-1-propene (1234ze-E) ; avantageusement ledit agent d'extraction organique peut avoir un facteur de séparation supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0.

Le facteur de séparation dudit agent d'extraction organique peut être différent suivant le couple binaire considéré. Ainsi, l'agent d'extraction organique peut être sélectionné de sorte à avoir un facteur de séparation supérieur ou égal à 1,4 sur base du couple binaire (1234yf/1243zf) et à avoir un facteur de séparation supérieur ou égal à 1,6 sur base du couple binaire (1234yf/1234zeE). Il en est de même pour la capacité d'absorption.

Ainsi, ledit agent d'extraction organique peut avoir une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité du 3,3,3-trifluoropropene (1243zf) dans ledit agent d'extraction organique à dilution infinie, avantageusement la capacité d'absorption C_{2,S} peut être supérieure ou égale à 0,40, de préférence supérieure ou égale à 0,60, plus préférentiellement supérieure ou égale à 0,80, en particulier supérieure ou égale à 1,0 ; et ledit agent d'extraction organique peut avoir une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité du trans-1,3,3,3-tetrafluoro-1-propene (1234ze-E) dans ledit agent d'extraction organique à dilution infinie, avantageusement la capacité d'absorption C_{2,S} est supérieure ou égale à 0,40, de préférence supérieure ou égale à 0,60, plus préférentiellement supérieure ou égale à 0,80, en particulier supérieure ou égale à 1,0.

Ainsi, ledit agent d'extraction organique peut avoir :
- un facteur de séparation S₁,₂ supérieur ou égal à 1,1, ledit facteur de séparation étant calculé par la formule S_{1,2} = (γ_{1,S}^{∗}P1)/(γ_{2,S}^{∗}P2) dans laquelle γ_{1,S} représente le coefficient d'activité du 2,3,3,3-tétrafluoro-1-propène dans ledit agent d'extraction organique à dilution infinie, P1 représente la pression de vapeur saturante du 2,3,3,3-tétrafluoro-1-propène, γ_{2,S} représente le coefficient d'activité du 3,3,3-trifluoropropene (1243zf) dans ledit agent d'extraction organique à dilution infinie et P2 représente la pression de vapeur saturante du 3,3,3-trifluoropropene (1243zf), avantageusement, ledit agent d'extraction organique peut avoir un facteur de séparation supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0 ;
- une capacité d'absorption C_{2,S} supérieure ou égale à 0,20, ladite capacité d'absorption étant calculé par la formule C₂,_{S} = 1/(yz,s) dans laquelle γ_{2,S} représente le coefficient d'activité du 3,3,3-trifluoropropene (1243zf) dans ledit agent d'extraction organique à dilution infinie, avantageusement la capacité d'absorption C_{2,S} peut être supérieure ou égale à 0,40, de préférence supérieure ou égale à 0,60, plus préférentiellement supérieure ou égale à 0,80, en particulier supérieure ou égale à 1,0 ;
- un facteur de séparation S_{1,2} supérieur ou égal à 1,1, ledit facteur de séparation étant calculé par la formule S_{1,2} = (γ_{1,S}^{∗}P1)/(γ_{2,S}^{∗}P2) dans laquelle γ_{1,S} représente le coefficient d'activité du 2,3,3,3-tétrafluoro-1-propène dans ledit agent d'extraction organique à dilution infinie, P1 représente la pression de vapeur saturante du 2,3,3,3-tétrafluoro-1-propène, γ_{2,S} représente le coefficient d'activité du trans-1,3,3,3-tetrafluoro-1-propene (1234ze-E) dans ledit agent d'extraction organique à dilution infinie et P2 représente la pression de vapeur saturante du trans-1,3,3,3-tetrafluoro-1-propene (1234ze-E) ; avantageusement ledit agent d'extraction organique peut avoir un facteur de séparation supérieur ou égal à 1,2, de préférence supérieur ou égal à 1,4, plus préférentiellement supérieur ou égal à 1,6, en particulier supérieur ou égal à 1,8, plus particulièrement supérieur ou égal à 2,0 ; et
- une capacité d'absorption C_{2,S} supérieure ou égale à 0,20, ladite capacité d'absorption étant calculé par la formule C_{2,S} = 1/(γ_{2,S}) dans laquelle γ_{2,S} représente le coefficient d'activité du trans-1,3,3,3-tetrafluoro-1-propene (1234ze-E) dans ledit agent d'extraction organique à dilution infinie, avantageusement la capacité d'absorption C_{2,S} est supérieure ou égale à 0,40, de préférence supérieure ou égale à 0,60, plus préférentiellement supérieure ou égale à 0,80, en particulier supérieure ou égale à 1,0.

Par exemple, ledit agent d'extraction organique peut être sélectionné de sorte à avoir un facteur de séparation supérieur ou égal à 1,2 et de sorte à avoir une capacité d'absorption supérieure ou égale à 0,4 pour les deux couples binaires 2,3,3,3-tétrafluoro-1-propène/trans-1,3,3,3-tetrafluoro-1-propene (1234ze-E) et 2,3,3,3-tétrafluoro-1-propène/3,3,3-trifluoropropene (1243zf). L'agent d'extraction de la présente invention est défini dans les revendications. Est également décrit un agent d'extraction qui peut être sélectionné parmi le groupe consistant en chloroethane, 1,1,1,3,3-pentafluoropropane, bromofluoromethane, 1-bromo-1,2-difluoroethylene, 1-chloro-1,1,2,2-tetrafluoropropane, acetaldehyde, 1,1,1-trifluoro-2-propanone, 2-chloro-1,1,1,2-tetrafluoropropane, 1,1,1,2,3-pentafluoropropane, 1-chloro-1,1-difluoropropane, 1,1,1-trifluoro-2-bromoethane, 1,1-dichloro-2,2,2-trifluoroethane, 1-chloro-2,2,3,3,3-pentafluoropropane, 1,2-dichloro-1,1,2-trifluoroethane, 2,2,2-trifluoroethylmethylether, furane, methylformate, isopropylamine, 1,1,2,2-tetrafluoroethylmethylether, 2-methoxy-1-propene, diethylether, 1,2-epoxypropane, ethanethiol, 2-chloro-2-fluoropropane, ethoxy-ethene, 1-chloro-2,2-difluoroethane, ethylmethylamine, dimethylsulfide, 2-chloropropane, bromoethane, dimethoxymethane, 2-chloro-1,1,1,3,3-pentafluoropropane, 1-chloro-1,2,2-trifluoropropane, 2,2-dichloro-1,1,1,3,3-pentafluoropropane, 2-amino-2-methylpropane, methylcyclopropylether, 3-chloropropene, 3-chloro-1,1,1-trifluoropropane, 1,1,1-trichloro-2,2,2-trifluoroethane, 1,2-dichloro-1,2-difluoroethane, 1,2-dichloro-1,1,2,3,3-pentafluoropropane, 1,1,2-trichloro-1,2,2-trifluoroethane, isopropylmethylamine, ethanedial, 1,2-dichloro-1,1,3,3,3-pentafluoropropane, 1-chloro-1,2,2,3-tetrafluoropropane, 1,1-dichloro-1,2,2,3,3-pentafluoropropane, 2-chloro-2-methylpropane, 3-chloro-1,1,1,3-tetrafluoropropane, 2,3-dichloro-1,1,1,2,3-pentafluoropropane, 1,1-dichloro-2,2,3,3,3-pentafluoropropane, 2-chloro-1,1,1,3-tetrafluoropropane, 2-propanethiol, 1,2-dichloro-3,3,3-trifluoropropene, 2-ethoxy-propane, 2,2-dichloro-1,1,1,3-tetrafluoropropane, 1-chloro-2,2-difluoropropane, methyl-t-butylether, diethylamine, propanone, methylacetate, ethyl1,1,2,2-tetrafluoroethylether, 4-methoxy-2-methyl-2-butanethiol, 2-bromopropane, 1,1-dichloro-2,2-difluoroethane, 2,2-dichloro-1,1,3,3-tetrafluoropropane, difluorodiethylsilane, 2,3-dichloro-1,1,1,2-tetrafluoropropane, 1,1-dichloro-1,2,2,3-tetrafluoropropane, tert-butylthiol, isobutanal, tetrahydrofurane, 1,3-dichloro-1,1,2,2-tetrafluoropropane, 1,1,1-trichloro-2,2-difluoroethane, 1,2-dichloro-1,2,3,3-tetrafluoropropane, 1-propanethiol, 2-chlorobutane, isopropylformate, diisopropylether, 1,3-dichloro-1,2,2,3-tetrafluoropropane, 1,2-dichloro-1,1,2,3-tetrafluoropropane, 2,3-dichloro-1,1,1,3-tetrafluoropropane, 1-bromopropane, 1,1-difluoro-1,2,2-trichloroethane, 1,1,2-trichloro-1,2-difluoroethane, methylglyoxal, 2-ethoxy-2-methyl-propane, 2-bromo-2-methylpropane, 1-chloro-3-fluoropropane, 1,1,1-trifluoro-2-propanol, 2,3-dichloro-1,1,1-trifluoropropane, ethylacetate, 1-chlorobutane, butanone, n-propylformate, 2-ethoxy-butane, pentafluoro-1-propanol, tert-butanol, 1,3-dichloro-1,1,2-trifluoropropane, 1-methoxy-2-methyl-butane, 1,1-dichloro-2,2,3-trifluoropropane, cyclohexene, 2,2-dimethoxypropane, 1,3,3-trichloro-1,1,2,2-tetrafluoropropane, 2-chloro-2-methylbutane, 1-ethoxy-2-methylpropane, diisopropylamine, 2-butanethiol, 1,1,1-trichloro-2,2,3,3-tetrafluoropropane, 1,2-dimethoxyethane, 1,1,3-trichloro-1,2,2,3-tetrafluoropropane, 1,3-dichloro-1,2,2-trifluoropropane, diethoxymethane, 2-methyl-1-propanethiol, isopropylacetate, 2-iodopropane, di-n-propylether, 2-bromobutane, diethylsulfide, 1-ethoxybutane, 1-fluorohexane, 1-methoxy-2-propanamine, 1,3-dichloro-1,2,3-trifluoropropane, 2-methoxyethanamine, 2-methylbutanal, tert-butylacetate, propionitrile, 3-chloropentane, 2-allyloxyethanol, butanethiol, isoamylchloride, 1-methoxy-pentane, ethylpropionate, 2-butanol, 1,2-dimethoxypropane, isopropyl-isobutyl-ether, 1,1,1-trichloro-2,2,3-trifluoropropane, 1-chloro-4-fluorobutane, 1-bromo-3-fluoropropane, 2,4,4-trimethyl-1-pentene, dioxane, 1-bromobutane, 3-pentanone, 1,1-diethoxyethane, 2-pentanone, 2-methyl-2-butanol, 2-methoxy-1propanamine, 1,1,3-trichloro-1,2,2-trifluoropropane, 1,1,3-trichloro-2,2,3-trifluoropropane, trimethoxymethane, 1,1-dichloro-2,2-difluoroethylmethylether, 2,2,4-trimethyl-2-pentene, 1,3-dioxane, 3,3-dimethyl-2-butanone, piperidine, 2-bromo-2-methylbutane, dipropylamine, 2,2,3,3-tetraflouro-1-propanol, 2-ethoxyethanamine, triethylfluorosilane, 1-methylcyclohexene, toluene, sec-butylacetate, 2-fluorotoluene, 2,2-dimethyl-1-propanol, n-methyl-1,2-ethanediamine, 2,2-diethoxypropane, 1,3,5-trioxane, 1-chloro-3,3-dimethylbutane, pyridine, 1,1,1,2-tetrachloro-3,3,3-trifluoropropane, n-methylmorpholine, 3-pentanol, 4-methyl-2-pentanone, 1,2-diaminoethane, isobutyl-tert-butylether, 2-bromopentane, butyronitrile, 1-butanol, sec-butyl-tert-butylether, 1-methoxy2-propanol, 1,1,3,3-tetrachloro-1,2,2-trifluoropropane, 1,2-propanediamine, 2,6-dimethyl-5-heptenal, 1,1,1,3-tetrachloro-2,2,3-trifluoropropane, 1-bromo-3-methylbutane, 1-(dimethylamino)-2-propanol, tetrahydrothiophene, 3-methyl-3-pentanol, 1,1-diethoxypropane, 2-methoxyethanol, 4,4,4-trifluorobutanol, diethylcarbonate, n-butylacetate, 1-pentanethiol, 2-chloro-1,1-dimethoxyethane, 2-hexanone, n-ethylethylenediamine, 5-hexen-2-one, 1,1-diethoxy-n,n-dimethylmethanamine, 2-methylpyridine, 1-bromopentane, 2-methoxy1-propanol, 1-ethoxy-2-propanol, hexanal, 4-methyl-2-pentanol, 1,2-octanediol, methoxycyclohexane, 2-(dimethylamino)-ethanol, cyclohexylamine, n-ethyl-2-dimethylaminoethylamine, ethoxyethanol, 3-hexanol, 2-hexanol, ethylbenzene, 2-methylpyrazine, 2-ethoxy-1-propanol, 1-pentanol, 1-methylpiperazine, n-ethyl-morpholine, 1,4-dimethylbenzene, 1,3-dimethylbenzene, 1,3-propanediamine, di-n-butylether, 3,3,4,4,5,5,6,6-octafluoro-1-pentanol, valeronitrile, (methyleneamino)acetonitrile, 2,3-dimethylbutanol, 1-ethoxy-hexane, 3-furfural, 2,6-dimethylpyridine, 4-methyl-2-hexanone, 1,2-dimethylbenzene, 1-methoxy-2-acetoxypropane, 1,1,1-triethoxyethane, styrene, 4-methylpyridine, n,n'-diethyl-1,2-ethanediamine, 2,6-dimethylmorpholine, 2-ethyl-1-butanol, 2-methyl-1-pentanol, methylhexanoate, 2-propoxyethanol, dimethylethanolamine, 1-propoxy-2-propanol.

Ledit agent d'extraction organique peut également être sélectionné de sorte à avoir un facteur de séparation supérieur ou égal à 1,4 et de sorte à avoir une capacité d'absorption supérieure ou égale à 0,6 pour les deux couples binaires 2,3,3,3-tétrafluoro-1-propène/trans-1,3,3,3-tetrafluoro-1-propene (1234ze-E) et 2,3,3,3-tétrafluoro-1-propène/3,3,3-trifluoropropene (1243zf). L'agent d'extraction de la présente invention est défini dans les revendications. Est également décrit un agent d'extraction qui peut être sélectionné parmi le groupe consistant en acetaldehyde, methylformate, 2-methoxy-1-propene, 1,2-epoxypropane, ethoxy-ethene, dimethoxymethane, 1-chloro-1,2,2-trifluoropropane, 3-chloro-1,1,1-trifluoropropane, ethanedial, 2-chloro-1,1,1,3-tetrafluoropropane, propanone, methylacetate, 4-methoxy-2-methyl-2-butanethiol, difluorodiethylsilane, isobutanal, isopropylformate, methylglyoxal, 2,3-dichloro-1,1,1-trifluoropropane, ethylacetate, butanone, n-propylformate, 1,3-dichloro-1,1,2-trifluoropropane, 1,1-dichloro-2,2,3-trifluoropropane, 1,2-dimethoxyethane, 1,3-dichloro-1,2,2-trifluoropropane, isopropylacetate, diethylsulfide, 1,3-dichloro-1,2,3-trifluoropropane, 2-methylbutanal, 2-allyloxyethanol, ethylpropionate, 1,2-dimethoxypropane, dioxane, 3-pentanone, 2-pentanone, trimethoxymethane, 1,3-dioxane, 3,3-dimethyl-2-butanone, pyridine, n-methylmorpholine, 4-methyl-2-pentanone, butyronitrile, 1-methoxy2-propanol, diethylcarbonate, n-butylacetate, 2-hexanone, 5-hexen-2-one, 2-methoxy1-propanol, 1-ethoxy-2-propanol, hexanal, 2-(dimethylamino)-ethanol, 2-methylpyrazine, 2-ethoxy-1-propanol, 1-methylpiperazine, n-ethyl-morpholine, valeronitrile, 4-methyl-2-hexanone, 1-methoxy-2-acetoxypropane, 2,6-dimethylmorpholine, methylhexanoate, 2-propoxyethanol, 1-propoxy-2-propanol.

En particulier, ledit agent d'extraction organique sélectionné peut avoir un facteur de séparation S_{1,2} supérieur ou égal à 1,4 et une capacité d'absorption C_{2,S} supérieure ou égale à 0,8 lorsqu'ils sont calculés pour le couple binaire 1234yf/1243zf ; et un facteur de séparation S_{1,2} supérieur ou égal à 1,6 et une capacité d'absorption C_{2,S} supérieure ou égale à 0,8 lorsqu'ils sont calculés pour le couple binaire 1234yf/1234zeE. L'agent d'extraction de la présente invention est défini dans les revendications. Est également décrit un agent d'extraction qui peut être choisi parmi le groupe consistant en 2-methoxy-1-propene,1,2-epoxypropane,ethoxy-ethene, dimethoxymethane, methylacetate, isobutanal, isopropylformate, ethylacetate, butanone, n-propylformate, 1,2-dimethoxyethane, isopropylacetate, 2-methylbutanal, ethylpropionate, 1,2-dimethoxypropane, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 2-hexanone, 5-hexen-2-one, 1-ethoxy-2-propanol, hexanal, 2-(dimethylamino)-ethanol, 2-methylpyrazine, 1-methylpiperazine, valeronitrile, 4-methyl-2-hexanone, 1-methoxy-2-acetoxypropane, 2,6-dimethylmorpholine, methylhexanoate, 1-propoxy-2-propanol. De préférence, ledit agent d'extraction organique peut être choisi parmi le groupe consistant en Ethoxy-ethene, dimethoxymethane, methylacetate, isobutanal, isopropylformate, ethylacetate, butanone, 1,2-dimethoxyethane, isopropylacetate, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 1-ethoxy-2-propanol, hexanal. En particulier, ledit agent d'extraction organique peut être sélectionné parmi le groupe consistant en dimethoxymethane, butanone, isopropylacetate, dioxane, trimethoxymethane, 1,3-dioxane, n-butylacetate, 1-ethoxy-2-propanol, hexanal. Plus particulièrement, ledit agent d'extraction organique peut être sélectionné parmi le groupe consistant en dimethoxymethane, isopropylacetate, dioxane, trimethoxymethane, 1,3-dioxane, n-butylacetate, 1-ethoxy-2-propanol, hexanal.

Selon un mode de réalisation préféré, ladite troisième composition peut être soumise à une distillation pour séparer d'une part l'agent d'extraction organique et d'autre part ledit au moins un des composés choisi parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf). Avantageusement, ladite troisième composition peut être soumise à une distillation pour séparer d'une part l'agent d'extraction organique et d'autre part 3,3,3-trifluoropropene (1243zf), trans-1,3,3,3-tetrafluoro-1-propene (1234ze-E) et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E).

Selon un mode de réalisation particulier, le courant comprenant le 2,3,3,3-tetrafluoropropène séparé à l'étape b) du présent procédé est purifié ultérieurement ou liquéfié et stocké. Dans ce mode de réalisation, des traces d'agent d'extraction organique peuvent être présentes dans le courant comprenant le 2,3,3,3-tetrafluoropropène. Les traces d'agent d'extraction organique dans ledit courant sont inférieures à 10 ppm, de préférence inférieures à 1 ppm, en particulier inférieures à 500 ppb, plus particulièrement inférieures à 100 ppb.

Le présent procédé permet donc de purifier le 2,3,3,3-tetrafluoro-1-propène. Avantageusement, la teneur en au moins un des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) dans le courant comprenant du 2,3,3,3-tetrafluoro-1-propène, obtenu à l'étape b) du présent procédé de purification est inférieure à la teneur de celui-ci ou de ceux-ci dans ladite première composition. Par exemple, la teneur en l'un quelconque des composés peut être diminuée de 50%, avantageusement de 75%, de préférence de 90%, en particulier de 95%, plus particulièrement de 98%. Avantageusement, la teneur en au moins deux, au moins trois, au moins quatre, au moins cinq, au moins six ou en l'ensemble desdits composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) peut être diminuée de 50%, avantageusement de 75%, de préférence de 90%, en particulier de 95%, plus particulièrement de 98%. De préférence, la teneur en 3,3,3-trifluoropropene (1243zf) et/ou trans-1,3,3,3-tetrafluoro-1-propene (1234ze-E) et/ou optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E) peut être diminuée de 50%, avantageusement de 75%, de préférence de 90%, en particulier de 95%, plus particulièrement de 98%.

Les teneurs sont exprimées en pourcentage en poids.

De préférence, le courant comprenant le 2,3,3,3-tetrafluoro-1-propène obtenu à l'étape b) du présent procédé de purification peut être dépourvu d'au moins un, d'au moins deux, d'au moins trois, d'au moins quatre, d'au moins cinq, d'au moins six ou de l'ensemble des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) lorsque celui-ci ou ceux-ci sont présents dans ladite première composition. En particulier, le courant comprenant le 2,3,3,3-tetrafluoro-1-propène obtenu à l'étape b) du présent procédé de purification peut être dépourvu de 3,3,3-trifluoropropene (1243zf) et/ou de trans-1,3,3,3-tetrafluoro-1-propene (1234ze-E) et/ou optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E). Le terme « dépourvu » signifie que le courant comprenant le 2,3,3,3-tetrafluoro-1-propène comprend moins de 50 ppm, avantageusement moins de 20 ppm, de préférence moins de 10 ppm du composé considéré sur base du poids total du courant.

Selon un mode de réalisation préféré, ladite première composition mise en oeuvre à l'étape a) du présent procédé peut être préalablement purifiée avant d'être utilisée. En effet, si ladite première composition comprend des impuretés ayant un point d'ébullition inférieur au point d'ébullition du 2,3,3,3-tetrafluoro-1-propène et optionnellement des impuretés lourdes, le présent procédé peut comprendre préalablement à l'étape a) les étapes :
i') mise en oeuvre ou fourniture d'une composition comprenant 2,3,3,3-tetrafluoro-1-propène, des impuretés ayant un point d'ébullition inférieur au point d'ébullition du 2,3,3,3-tetrafluoro-1-propène, et au moins un des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf), et optionnellement des impuretés lourdes ; de préférence 2,3,3,3-tetrafluoro-1-propène, des impuretés ayant un point d'ébullition inférieur au point d'ébullition du 2,3,3,3-tetrafluoro-1-propène, 3,3,3-trifluoropropene (1243zf), trans-1,3,3,3-tetrafluoro-1-propene (1234ze-E) et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E), et optionnellement des impuretés lourdes ;
ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, et au moins un des composés choisis parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf), et optionnellement des impuretés lourdes, récupéré en bas de colonne de distillation ; avantageusement le premier courant comprend 2,3,3,3-tetrafluoro-1-propène, 3,3,3-trifluoropropene (1243zf), trans-1,3,3,3-tetrafluoro-1-propene (1234ze-E) et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E) ;
iii') optionnellement, distillation dudit premier courant récupéré en bas de colonne de distillation à l'étape ii') pour récupérer en tête de colonne un second courant comprenant du 2,3,3,3-tetrafluoro-1-propène, et au moins un des composés choisis parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) et en bas de colonne de distillation un courant comprenant les impuretés lourdes ; avantageusement le second courant comprend 2,3,3,3-tetrafluoro-1-propène, 3,3,3-trifluoropropene (1243zf), trans-1,3,3,3-tetrafluoro-1-propene (1234ze-E) et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E) ;
ledit premier courant récupéré à l'étape ii') ou ledit second courant récupéré à l'étape iii') corresponde à ladite première composition mise en oeuvre à l'étape a).

Lesdites impuretés ayant un point d'ébullition inférieur au point d'ébullition du 2,3,3,3-tetrafluoro-1-propène peuvent être trifluorométhane (F23), monofluorométhane (F41), difluorométhane (F32), pentafluoroéthane (F125), 1,1,1-trifluoroéthane (F143a), trifluoropropyne ou 1-chloro-pentafluoroéthane (F115). Les impuretés lourdes peuvent contenir par exemple 1,1,1,3,3,3-hexafluoropropane (236fa), 1,1,1,2,3,3-hexafluoropropane (236ea), 1,1,1,2,3,3,3-heptafluoropropane (227ca), cis-1,3,3,3-tetrafluoro-1-propene (1234ze-Z), des dimères ou des trimères issus de l'un des composés présents dans la composition ou le courant considéré, par exemple des hydrocarbures en C4 ou C5 tels que hexafluorobutène (1336), heptafluorobutène (1327), octafluorobutane (338), nonafluoropentène (1429), heptafluoropentène (1447).

Selon un second aspect, la présente invention fournit un procédé de production du 2,3,3,3-tetrafluoro-1-propène. En outre, ce procédé peut inclure sa purification. Ainsi, la présente invention fournit un procédé de production de 2,3,3,3-tetrafluoro-1-propène comprenant les étapes de :
A) fluoration en présence d'un catalyseur d'un composé de formule CX(Y)₂-CX(Y)ₘ-CHₘXY (I) dans laquelle X et Y représentent indépendamment H, F, ou Cl et m = 0 ou 1 ; et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;
B) récupération d'un courant comprenant du 2,3,3,3-tetrafluoro-1-propène, et au moins un des composés choisis parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) ; avantageusement un courant comprenant du 2,3,3,3-tetrafluoro-1-propène, 3,3,3-trifluoropropene (1243zf), trans-1,3,3,3-tetrafluoro-1-propene (1234ze-E) et optionnellement ou non au moins un des composés choisis parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E) ;
C) mise en oeuvre du procédé de purification du 2,3,3,3-tetrafluoro-1-propène selon la présente invention à partir du courant récupéré à l'étape B).

Selon un mode de réalisation préféré, le procédé de production de 2,3,3,3-tetrafluoro-1-propène comprenant les étapes de :
A) fluoration en présence d'un catalyseur d'un composé de formule CX(Y)₂-CX(Y)ₘ-CHₘXY (I) dans laquelle X et Y représentent indépendamment H, F, ou Cl et m = 0 ou 1 ; et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;
B) récupération d'un courant comprenant du 2,3,3,3-tetrafluoro-1-propène, et au moins un des composés choisis parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) ; de préférence un courant comprenant du 2,3,3,3-tetrafluoro-1-propène, 3,3,3-trifluoropropene (1243zf), trans-1,3,3,3-tetrafluoro-1-propene (1234ze-E) et optionnellement ou non au moins un des composés choisis parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E) ;
C) mise en oeuvre du procédé de purification du 2,3,3,3-tetrafluoro-1-propène à partir du courant récupéré à l'étape B) tel que défini dans les revendications et comprenant les étapes de :
   a) mise en contact de ladite première composition avec au moins un agent d'extraction organique pour former une seconde composition ;
   b) distillation extractive de ladite seconde composition pour former :
      i) une troisième composition comprenant ledit agent d'extraction organique et ledit au moins un des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) ; de préférence une troisième composition comprenant ledit agent d'extraction organique, 3,3,3-trifluoropropene (1243zf), trans-1,3,3,3-tetrafluoro-1-propene (1234ze-E) et optionnellement ou non au moins un des composés choisis parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E) ; et
      ii) un courant comprenant le 2,3,3,3-tétrafluoro-1-propène,
   c) récupération et séparation de ladite troisième composition pour former un courant comprenant ledit agent d'extraction organique et un courant comprenant ledit au moins un des composés sélectionné parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf); de préférence un courant comprenant ledit agent d'extraction organique et un courant comprenant 3,3,3-trifluoropropene (1243zf), trans-1,3,3,3-tetrafluoro-1-propene (1234ze-E) et optionnellement ou non au moins un des composés choisis parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E).

De préférence, le courant comprenant ledit agent d'extraction organique est recyclé à l'étape a).

Selon un mode de réalisation particulier, le courant récupéré à l'étape B) du procédé de production du 2,3,3,3-tétrafluoro-1-propène peut comprendre de l'acide fluorhydrique. Dans ce cas, préalablement à l'étape C), le courant récupéré à l'étape B) peut subir une distillation préalable B1') pour éliminer HF en bas de colonne de distillation. Un courant comprenant 2,3,3,3-tétrafluoro-1-propène, et au moins un des composés choisi parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) est récupéré en tête de colonne de distillation, de préférence un courant comprenant du 2,3,3,3-tetrafluoro-1-propène, 3,3,3-trifluoropropene (1243zf), trans-1,3,3,3-tetrafluoro-1-propene (1234ze-E) et optionnellement ou non au moins un des composés choisis parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E). Ce dernier courant récupéré en tête de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut comprendre des impuretés ayant un point d'ébullition inférieur au point d'ébullition du 2,3,3,3-tetrafluoro-1-propène. Dans ce cas, préalablement à l'étape C), le courant récupéré à l'étape B) peut subir une distillation préalable B2'), subséquente ou non à l'étape B1'), pour éliminer en tête de colonne de distillation lesdites impuretés ayant un point d'ébullition inférieur au point d'ébullition du 2,3,3,3-tetrafluoro-1-propène et former un premier courant comprenant 2,3,3,3-tetrafluoro-1-propène, et au moins un des composés choisi parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) récupéré en bas de colonne de distillation ; de préférence un courant comprenant du 2,3,3,3-tetrafluoro-1-propène, 3,3,3-trifluoropropene (1243zf), trans-1,3,3,3-tetrafluoro-1-propene (1234ze-E) et optionnellement ou non au moins un des composés choisis parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E). Ce dernier courant récupéré en bas de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut comprendre des impuretés lourdes. Dans ce cas, préalablement à l'étape C), le courant récupéré à l'étape B) peut subir une distillation préalable B3'), subséquente ou non à l'étape B1') et/ou B2'), pour éliminer en bas de colonne de distillation lesdites impuretés lourdes ; et former un premier courant comprenant 2,3,3,3-tetrafluoro-1-propène, et au moins un des composés choisi parmi le groupe consistant en chlorométhane (40), 1,1-difluoroéthane (152a), chloropentafluoroéthane (115), 1,1,1,2-tetrafluoroethane (134a), trans-1,3,3,3-tetrafluoro-1-propene (1234ze-E), trans-1,2,3,3,3-pentafluoropropene (1225ye-E) et 3,3,3-trifluoropropene (1243zf) récupéré en tête de colonne de distillation ; de préférence un premier courant comprenant du 2,3,3,3-tetrafluoro-1-propène, 3,3,3-trifluoropropene (1243zf), trans-1,3,3,3-tetrafluoro-1-propene (1234ze-E) et optionnellement ou non au moins un des composés choisis parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E). Ce dernier courant récupéré en tête de colonne de distillation est ensuite soumis à l'étape C) du procédé de production du 2,3,3,3-tetrafluoro-1-propène.

Selon un mode de réalisation particulier, le courant récupéré à l'étape B) du procédé de production du 2,3,3,3-tétrafluoro-1-propène peut également comprendre HCl. L'acide chlorhydrique peut être récupéré par distillation avant ou après l'étape B1' indépendamment des autres étapes du procédé.

Plus particulièrement, l'étape A) est effectuée à partir de 1,1,2,3-tetrachloropropène, le 2,3,3,3,-tetrachloropropène, le 1,1,3,3-tetrachloropropène, le 1,3,3,3-tetrachloropropène, le 1,1,1,2,3-pentachloropropane, le 1,1,1,3,3-pentachloropropane, le 1,1,2,2,3-pentachloropropane, le 1,2-dichloro-3,3,3-trifluoropropane, le 2-chloro-2,3,3,3-tetrafluoropropane, le 1,1,1,2,2-pentafluoropropane, le 1-chloro-1,3,3,3-tetrafluoropropane et le 1,1,1,3,3-pentafluoropropane, de préférence à partir de 1,1,1,2,3-pentachloropropane, 1,1,2,3,tetrachloropropène, du 1,1,1,2,2-pentafluoropropane et/ou du 2-chloro-3,3,3-trifluoro-1-propène ; en particulier à partir du 1,1,1,2,3-pentachloropropane (240db).

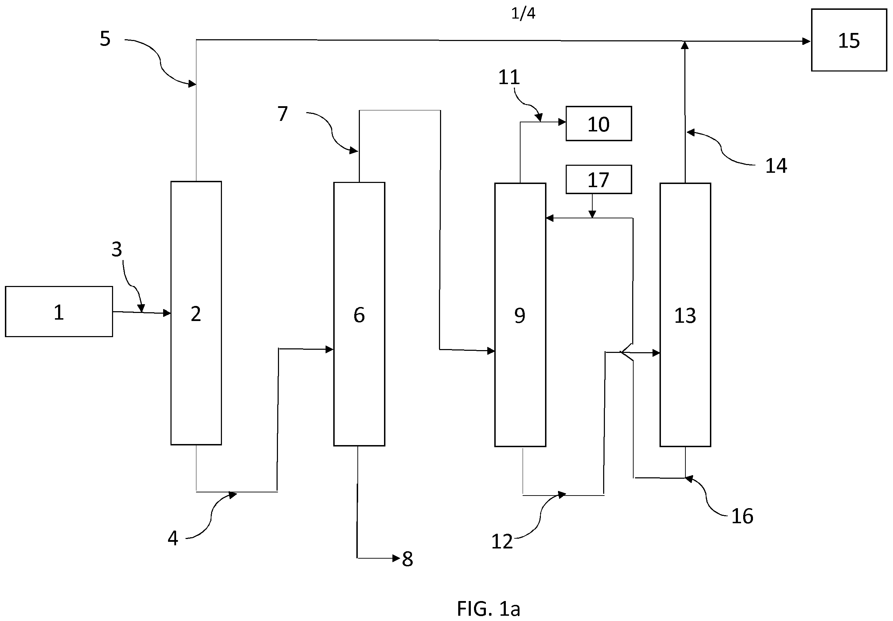

La figure 1a représente un schéma simplifié d'un dispositif mettant en oeuvre un procédé de purification du 2,3,3,3-tetrafluoro-1-propene selon un mode de réalisation particulier de l'invention. Le mélange issu de la réaction de fluoration, en présence d'un catalyseur, d'un composé de formule (CXₙY₃₋ₙ)CHXCHₘ₊₁X₂₋ₘ (I) et/ou de fluoration catalytique, en présence d'un catalyseur, d'un composé de formule (CXₙY₃₋ₙ)CHₚX₁₋ₚ=CHₘX₂₋ₘ (II) est obtenu en 1. Le mélange comprend, dans ce mode de réalisation particulier, 2,3,3,3-tetrafluoro-1-propene, 3,3,3-trifluoropropene (1243zf), trans-1,3,3,3-tetrafluoro-1-propene (1234ze-E) et optionnellement au moins un des composés choisis parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E), des impuretés ayant un point d'ébullition inférieur au point d'ébullition du 2,3,3,3-tetrafluoro-1-propene et des impuretés lourdes. Le mélange est transféré dans une colonne de distillation 2 par le conduit 3. Des impuretés sont récupérées en tête de la colonne de distillation 2 et acheminées par le conduit 5 à un incinérateur ou un dispositif de purification 15. Le résidu obtenu en bas de colonne de distillation et comprenant les autres constituants du mélange est acheminé vers une seconde colonne de distillation 6 par le conduit 4. La distillation effectuée en 6 vise à séparer les impuretés lourdes des autres constituants du mélange. Les conditions opératoires de distillation sont donc adaptées à ce but. Les impuretés lourdes sont récupérées en bas de colonne de distillation 8 et les autres constituants sont récupérés en tête de colonne de distillation et acheminés vers la colonne de distillation extractive 9 via le conduit 7. La distillation extractive effectuée en 9 vise à séparer le 2,3,3,3-tetrafluoro-1-propene des autres constituants du mélange mentionnés ci-dessus. Le dispositif de distillation extractive 9 est alimenté par l'agent d'extraction organique 17 sélectionné selon la méthode décrite dans la présente demande. Le 2,3,3,3-tetrafluoro-1-propene est récupéré en tête du dispositif de distillation extractive 9 pour être stocké ou purifié en 10 via le conduit 11. Le mélange récupéré en bas du dispositif de distillation extractive 9 comprend notamment l'agent d'extraction organique 17, 3,3,3-trifluoropropene (1243zf), trans-1,3,3,3-tetrafluoro-1-propene (1234ze-E) et optionnellement au moins un des composés suivants chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E). Le mélange récupéré en bas du dispositif de distillation extractive 9 est acheminé par le conduit 12 vers un dispositif de distillation 13 visant à séparer l'agent d'extraction organique des autres composés présents. L'agent d'extraction organique est récupéré en bas du dispositif de distillation et recyclé par le conduit 16 vers le dispositif de distillation extractive 9. Les composés récupérés en tête du dispositif de distillation 13 sont acheminés par le conduit 14 vers le conduit 5 pour être incinérés ou purifiés en 15.

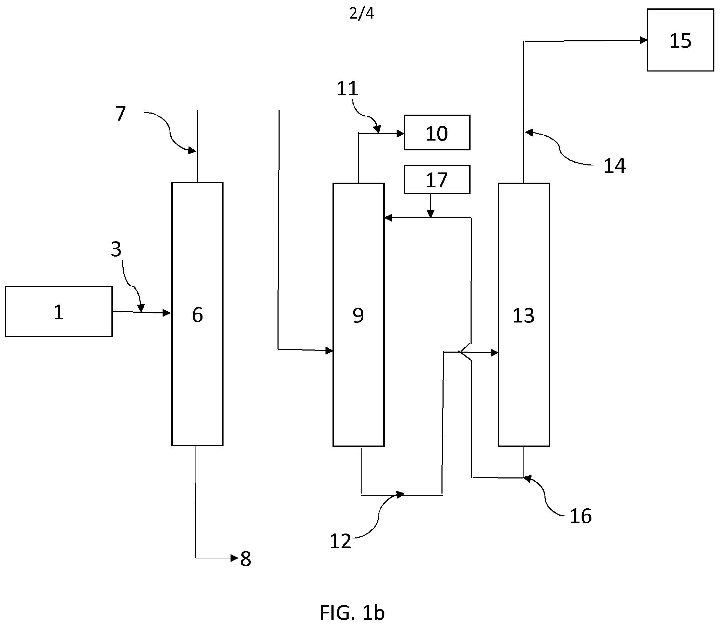

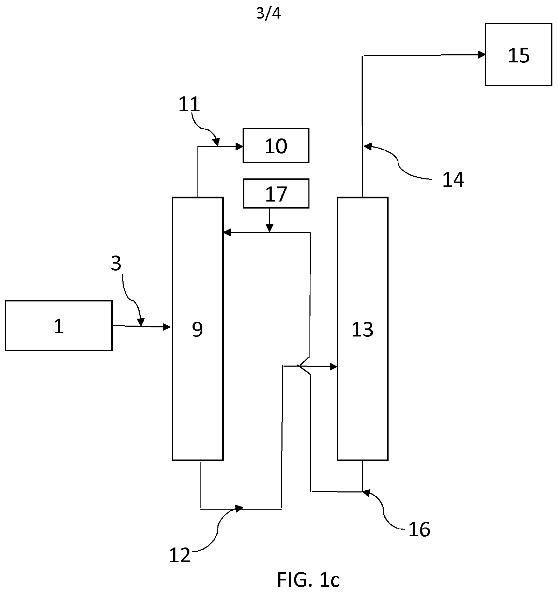

Le mélange fourni en 1 peut être dépourvu d'impuretés ayant un point d'ébullition inférieur à celui du 2,3,3,3-tetrafluoro-1-propène. Dans ce cas, comme illustré à la Fig. 1b, le mélange 1 est acheminé par le conduit 3 vers la colonne de distillation 6 pour être traité comme expliqué ci-dessus en relation avec la Fig. 1a. Dans un autre mode de réalisation particulier illustré à la Fig. 1c, le mélange 1 peut être dépourvu d'impuretés ayant un point d'ébullition inférieur à celui du 2,3,3,3-tetrafluoro-1-propène et d'impuretés lourdes. Dans ce cas, le mélange est acheminé directement vers la colonne de distillation extractive 9 pour y être traité comme expliqué ci-dessus en relation avec la Fig. 1a.

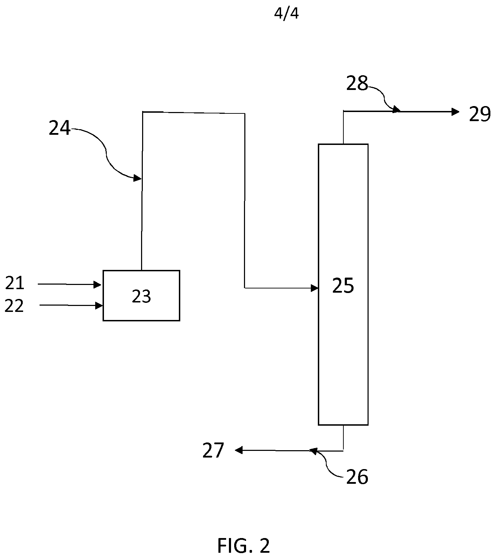

La Fig. 2 illustre schématiquement un dispositif mettant en oeuvre un procédé de production du 2,3,3,3-tetrafluoropropène selon un mode de réalisation particulier de la présente invention. L'acide fluorhydrique 21 est mis en contact avec du 1,1,1,2,3-pentachloropropane (240db) 22 dans un réacteur 23. Le mélange obtenu comprend HF, HCl, 2,3,3,3-tetrafluoro-1-propène, 3,3,3-trifluoropropene (1243zf), trans-1,3,3,3-tetrafluoro-1-propene (1234ze-E), et optionnellement ou non au moins un des composés suivants chlorométhane (40), 1,1-difluoroéthane (152a), chloropentafluoroéthane (115), 1,1,1,2-tetrafluoroethane (134a), trans-1,2,3,3,3-pentafluoropropene (1225ye-E) ; et optionnellement ou non des impuretés lourdes ou ayant un point d'ébullition inférieur à celui du 2,3,3,3-tetrafluoro-1-propène. Le mélange est récupéré en sortie de réacteur et acheminé vers une colonne de distillation 25 par le conduit 24. Le courant obtenu en bas de colonne de distillation comprenant HF et éventuellement des impuretés lourdes est acheminé vers le dispositif de purification 27 via le conduit 26 pour purifier HF qui sera recyclé éventuellement en 23. Les autres constituants du mélange sont acheminés via le conduit 28 vers un dispositif de purification 29 de purification du 2,3,3,3-tetrafluoro-1-propène. Le dispositif de purification 29 peut être l'un quelconque des dispositifs illustrés aux Fig. 1a-1b.

Le catalyseur utilisé dans le présent procédé de production de 2,3,3,3-tétrafluoropropène peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document EP 939071 (paragraphe [0022]), au document WO 2008/054781 (en p.9 I.22-p.10 I.34), et au document WO 2008/040969 (revendication 1), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de nickel.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :
- avec un rapport molaire HF / composé de formule (I) et/ou (II) de 3:1 à 150:1, de préférence de 4:1 à 125:1 et de manière plus particulièrement préférée de 5:1 à 100:1 ;
- avec un temps de contact de 3 à 100 s, de préférence 4 à 75 s et plus particulièrement 5 à 50 s (volume de catalyseur divisé par le flux entrant total, ajusté à la température et à la pression de fonctionnement) ;
- à une pression allant de la pression atmosphérique à 20 bar, de préférence de 2 à 18 bar et plus particulièrement de 3 à 15 bars;
- à une température (température du lit de catalyseur) de 200 à 450°C, de préférence de 250 à 400°C, et plus particulièrement de 280 à 380°C.

La durée de l'étape de réaction est typiquement de 10 à 8000 heures, de préférence de 50 à 5000 heures et de manière plus particulièrement préférée de 70 à 1000 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

### Méthode de sélection de l'agent d'extraction organique

L'agent d'extraction de la présente invention est défini dans les revendications.

La sélection de l'agent d'extraction organique est déterminée par l'utilisation du modèle Cosmo-RS implémenté dans le logiciel COSMOTHERM. Pour ce couple binaire sélectionné, un facteur de séparation est calculé pour chacun des solvants étudiés par l'équation suivante :
S_{1,2} = (γ_{1,S}^{∗}P1)/(γ_{2,S}^{∗}P2) dans laquelle
γ_{1,S} représente le coefficient d'activité du premier composé 1 dans l'agent d'extraction organique considéré à dilution infinie,
P1 représente la pression de vapeur saturante du premier composé 1,
γ_{2,S} représente le coefficient d'activité du second composé 2 du couple binaire dans l'agent d'extraction organique considéré à dilution infinie,
P2 représente la pression de vapeur saturante du second composé.

Une capacité d'absorption est également calculée pour chacun des solvants étudiés et pour un couple binaire (1,2) considéré. La capacité d'absorption est calculée par la formule C_{2,S} = 1/(γ_{2,S}) dans laquelle γ_{2,S} représente le coefficient d'activité du second composé du couple binaire considéré dans ledit agent d'extraction organique étudié à dilution infinie. Les calculs sont répétés pour chaque agent d'extraction organique étudié. Des valeurs minimales de facteur de séparation et de capacité d'absorption sont identifiées afin de permettre une séparation suffisante entre le premier composé et le second composé du couple binaire (1,2) considéré. La pression de vapeur saturante est considérée pour une température de 25°C.

### Exemple

Pour purifier le 2,3,3,3-tetrafluoro-1-propene, le couple binaire 2,3,3,3-tetrafluoro-1-propene/3,3,3-trifluoropropene (1243zf) et le couple binaire 2,3,3,3-tetrafluoro-1-propene/ trans-1,3,3,3-tetrafluoro-1-propene (1234ze-E) ont été considérés pour sélectionner l'agent d'extraction organique. Sur base des informations obtenues par le modèle Cosmo-RS, les solvants repris dans le tableau 1 ci-dessous ont été testés pour la distillation extractive d'un mélange comprenant 2,3,3,3-tetrafluoro-1-propene, 3,3,3-trifluoropropene (1243zf) et trans-1,3,3,3-tetrafluoro-1-propene (1234ze-E).

**Tableau 1 - Capacité et Facteur de séparation de l'agent d'extraction organique**

| Agent extraction organique | Capacité d'absorption (1234yf/1243zf) | Facteur de séparation (1234yf/1243zf) | Capacité d'absorption (1234yf/1234zeE) | Facteur de séparation (1234yf/1243zeE) |
|---|---|---|---|---|
| Dimethoxymethane | 1,19 | 1,44 | 1,37 | 1,95 |
| isopropylacetate | 1,10 | 1,42 | 1,42 | 2,15 |
| dioxane | 0,92 | 1,59 | 1,09 | 2,24 |
| trimethoxymethane | 1,11 | 1,54 | 1,28 | 2,11 |
| 1,3-dioxane | 0,95 | 1,64 | 1,09 | 2,23 |
| n-butylacetate | 1,05 | 1,41 | 1,35 | 2,14 |
| 1-ethoxy-2-propanol | 0,85 | 1,45 | 1,12 | 2,27 |
| hexanal | 0,93 | 1,43 | 1,09 | 1,98 |

Les résultats ont été confirmés à partir d'un mélange comprenant 92.0 % en poids de 2,3,3,3-tetrafluoro-1-propene, 7,5% en poids de trans-1,3,3,3-tetrafluoro-1-propene (1234ze-E) et 0.5 % en poids de 3,3,3-trifluoropropene (1243zf) sur base du poids total du mélange des trois composés. Le reste de la composition est formée par l'agent d'extraction organique testé.

## Revendications

1. Procédé de purification du 2,3,3,3-tétrafluoro-1-propène (1234yf) à partir d'une première composition comprenant du 2,3,3,3-tétrafluoro-1-propène, 3,3,3-trifluoropropene (1243zf), trans-1,3,3,3-tetrafluoro-1-propene (1234ze-E) et optionnellement ou non au moins un composé sélectionné parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), ledit procédé comprenant les étapes de:
a) mise en contact de ladite première composition avec au moins un agent d'extraction organique pour former une seconde composition ;
b) distillation extractive de ladite seconde composition pour former :
i) une troisième composition comprenant ledit agent d'extraction organique, 3,3,3-trifluoropropene (1243zf), trans-1,3,3,3-tetrafluoro-1-propene (1234ze-E) et optionnellement ou non au moins un composé sélectionné parmi le groupe consistant en chlorométhane (40), 1,1-difluoroéthane (152a), chloropentafluoroéthane (115), 1,1,1,2-tetrafluoroethane (134a) et trans-1,2,3,3,3-pentafluoropropene (1225ye-E); et
ii) un courant comprenant le 2,3,3,3-tétrafluoro-1-propène,
c) récupération et séparation de ladite troisième composition pour former un courant comprenant ledit agent d'extraction organique et un courant comprenant 3,3,3-trifluoropropene (1243zf), trans-1,3,3,3-tetrafluoro-1-propene (1234ze-E) et optionnellement ou non au moins un composé sélectionné parmi le groupe consistant en chlorométhane (40), 1,1-difluoroéthane (152a), chloropentafluoroéthane (115), 1,1,1,2-tetrafluoroethane (134a), et trans-1,2,3,3,3-pentafluoropropene (1225ye-E) ; de préférence le courant comprenant ledit agent d'extraction organique est recyclé à l'étape a) ;
**caractérisé en ce que** ledit agent d'extraction organique est choisi parmi le groupe consistant en acetaldehyde, methylformate, 2-methoxy-1-propene, 1,2-epoxypropane, ethoxy-ethene, dimethoxymethane, 1-chloro-1,2,2-trifluoropropane, 3-chloro-1,1,1-trifluoropropane, ethanedial, 2-chloro-1,1,1,3-tetrafluoropropane, propanone, methylacetate, 4-methoxy-2-methyl-2-butanethiol, difluorodiethylsilane, isobutanal, isopropylformate, methylglyoxal, 2,3-dichloro-1,1,1-trifluoropropane, ethylacetate, butanone, n-propylformate, 1,3-dichloro-1,1,2-trifluoropropane, 1,1-dichloro-2,2,3-trifluoropropane, 1,2-dimethoxyethane, 1,3-dichloro-1,2,2-trifluoropropane, isopropylacetate, diethylsulfide, 1,3-dichloro-1,2,3-trifluoropropane, 2-methylbutanal, 2-allyloxyethanol, ethylpropionate, 1,2-dimethoxypropane, dioxane, 3-pentanone, 2-pentanone, trimethoxymethane, 1,3-dioxane, 3,3-dimethyl-2-butanone, pyridine, n-methylmorpholine, 4-methyl-2-pentanone, butyronitrile, 1-methoxy2-propanol, diethylcarbonate, n-butylacetate, 2-hexanone, 5-hexen-2-one, 2-methoxyl-propanol, 1-ethoxy-2-propanol, hexanal, 2-(dimethylamino)-ethanol, 2-methylpyrazine, 2-ethoxy-1-propanol, 1-methylpiperazine, n-ethyl-morpholine, valeronitrile, 4-methyl-2-hexanone, 1-methoxy-2-acetoxypropane, 2,6-dimethylmorpholine, methylhexanoate, 2-propoxyethanol, 1-propoxy-2-propanol.

2. Procédé selon l'une quelconque des revendications précédentes **caractérisé en ce que** ladite première composition est une composition azéotropique ou quasi-azéotropique comprenant 2,3,3,3-tetrafluoro-1-propène, trans-1,3,3,3-tetrafluoro-1-propene (1234ze-E), 3,3,3-trifluoropropene (1243zf) et optionnellement ou non au moins un des composés sélectionné parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E).

3. Procédé selon l'une quelconque des revendications précédentes **caractérisé en ce que** ledit agent d'extraction organique est choisi parmi le groupe consistant en 2-methoxy-1-propene, 1,2-epoxypropane, ethoxy-ethene, dimethoxymethane, methylacetate, isobutanal, isopropylformate, ethylacetate, butanone, n-propylformate, 1,2-dimethoxyethane, isopropylacetate, 2-methylbutanal, ethylpropionate, 1,2-dimethoxypropane, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 2-hexanone, 5-hexen-2-one, 1-ethoxy-2-propanol, hexanal, 2-(dimethylamino)-ethanol, 2-methylpyrazine, 1-methylpiperazine, valeronitrile, 4-methyl-2-hexanone, 1-methoxy-2-acetoxypropane, 2,6-dimethylmorpholine, methylhexanoate, 1-propoxy-2-propanol ; avantageusement ledit agent d'extraction organique est choisi parmi le groupe consistant en éthoxy-ethene, dimethoxymethane, methylacetate, isobutanal, isopropylformate, ethylacetate, butanone, 1,2-dimethoxyethane, isopropylacetate, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 1-ethoxy-2-propanol, hexanal ; de préférence ledit agent d'extraction organique est choisi parmi le groupe consistant en diméthoxymethane, butanone, isopropylacetate, dioxane, trimethoxymethane, 1,3-dioxane, n-butylacetate, 1-ethoxy-2-propanol, hexanal ; en particulier ledit agent d'extraction organique est choisi parmi le groupe consistant en diméthoxymethane, isopropylacetate, dioxane, trimethoxymethane, 1,3-dioxane, n-butylacetate, 1-ethoxy-2-propanol, hexanal.

4. Procédé selon l'une quelconque des revendications précédentes **caractérisé en ce que** le courant comprenant le 2,3,3,3-tetrafluoro-1-propène formé à l'étape b) est récupéré et est dépourvu de trans-1,3,3,3-tetrafluoro-1-propene (1234ze-E) et/ou de 3,3,3-trifluoropropene (1243zf) et/ou d'au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E).

5. Procédé selon l'une quelconque des revendications précédentes **caractérisé en ce que** ladite première composition comprend des impuretés ayant un point d'ébullition inférieur au point d'ébullition du 2,3,3,3-tetrafluoro-1-propène et optionnellement des impuretés lourdes, le procédé comprenant préalablement à l'étape a) les étapes :
i') mise en oeuvre ou fourniture d'une composition comprenant 2,3,3,3-tetrafluoro-1-propène, des impuretés ayant un point d'ébullition inférieur au point d'ébullition du 2,3,3,3-tetrafluoro-1-propène, 3,3,3-trifluoropropene (1243zf), trans-1,3,3,3-tetrafluoro-1-propene (1234ze-E),et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), et optionnellement des impuretés lourdes ;
ii') distillation de ladite composition de l'étape i) pour éliminer en tête de colonne des impuretés ayant un point d'ébullition inférieur au point d'ébullition du 2,3,3,3-tetrafluoro-1-propène et former un premier courant comprenant 2,3,3,3-tetrafluoro-1-propène, 3,3,3-trifluoropropene (1243zf), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), et optionnellement des impuretés lourdes ; récupéré en bas de colonne de distillation ;
iii') optionnellement, distillation dudit premier courant récupéré en bas de colonne de distillation à l'étape ii') pour récupérer en tête de colonne un second courant comprenant du 2,3,3,3-tetrafluoro-1-propène, 3,3,3-trifluoropropene (1243zf), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E), et en bas de colonne de distillation un courant comprenant les impuretés lourdes ;
ledit premier courant récupéré à l'étape ii') ou ledit second courant récupéré à l'étape iii') correspondent à ladite première composition mise en oeuvre à l'étape a).

6. Procédé de production et de purification du 2,3,3,3-tetrafluoro-1-propène comprenant les étapes de :
A) fluoration en présence d'un catalyseur d'un composé de formule (I) CX(Y)₂-CX(Y)ₘ-CHₘXY dans laquelle X et Y représentent indépendamment un atome d'hydrogène, de fluor ou de chlore et m = 0 ou 1 et/ou fluoration en présence d'un catalyseur d'un composé de formule (CXₙY₃₋ₙ)CHₚX₁₋ₚCHₘX₂₋ₘ (II) dans lequel X est indépendamment les uns des autres Cl, F, I ou Br ; Y est indépendamment les uns des autres H, Cl, F, I ou Br ; n est 1, 2 ou 3 ; et m est 0, 1 ou 2 ; et p est 0 ou 1 ;
B) récupération d'un courant comprenant du 2,3,3,3-tetrafluoro-1-propène, 3,3,3-trifluoropropene (1243zf), trans-1,3,3,3-tetrafluoro-1-propene (1234ze-E), et optionnellement ou non au moins un des composés sélectionnés parmi le groupe consistant en chlorométhane (40), 1,1-difluoroéthane (152a), chloropentafluoroéthane (115), 1,1,1,2-tetrafluoroethane (134a), trans-1,2,3,3,3-pentafluoropropene (1225ye-E) ;
C) mise en oeuvre du procédé selon l'une quelconque des revendications 1 à 5 à partir du courant récupéré à l'étape B).

7. Composition comprenant du 2,3,3,3-tetrafluoropropène, 3,3,3-trifluoropropene (1243zf), trans-1,3,3,3-tetrafluoro-1-propene (1234ze-E) et un agent d'extraction organique sélectionné parmi le groupe consistant en acétaldehyde, methylformate, 2-methoxy-1-propene, 1,2-epoxypropane, ethoxy-ethene, dimethoxymethane, 1-chloro-1,2,2-trifluoropropane, 3-chloro-1,1,1-trifluoropropane, ethanedial, 2-chloro-1,1,1,3-tetrafluoropropane, propanone, methylacetate, 4-methoxy-2-methyl-2-butanethiol, difluorodiethylsilane, isobutanal, isopropylformate, methylglyoxal, 2,3-dichloro-1,1,1-trifluoropropane, ethylacetate, butanone, n-propylformate, 1,3-dichloro-1,1,2-trifluoropropane, 1,1-dichloro-2,2,3-trifluoropropane, 1,2-dimethoxyethane, 1,3-dichloro-1,2,2-trifluoropropane, isopropylacetate, diethylsulfide, 1,3-dichloro-1,2,3-trifluoropropane, 2-methylbutanal, 2-allyloxyethanol, ethylpropionate, 1,2-dimethoxypropane, dioxane, 3-pentanone, 2-pentanone, trimethoxymethane, 1,3-dioxane, 3,3-dimethyl-2-butanone, pyridine, n-methylmorpholine, 4-methyl-2-pentanone, butyronitrile, 1-methoxy2-propanol, diethylcarbonate, n-butylacetate, 2-hexanone, 5-hexen-2-one, 2-methoxyl-propanol, 1-ethoxy-2-propanol, hexanal, 2-(dimethylamino)-ethanol, 2-methylpyrazine, 2-ethoxy-1-propanol, 1-methylpiperazine, n-ethyl-morpholine, valeronitrile, 4-methyl-2-hexanone, 1-methoxy-2-acetoxypropane, 2,6-dimethylmorpholine, methylhexanoate, 2-propoxyethanol, 1-propoxy-2-propanol.

8. Composition selon la revendication précédente **caractérisée en ce que** ledit agent d'extraction organique est sélectionné parmi le groupe consistant en 2-methoxy-1-propene, 1,2-epoxypropane, ethoxy-ethene, dimethoxymethane, methylacetate, isobutanal, isopropylformate, ethylacetate, butanone, n-propylformate, 1,2-dimethoxyethane, isopropylacetate, 2-methylbutanal, ethylpropionate, 1,2-dimethoxypropane, dioxane, 3-pentanone, 2-pentanone, trimethoxymethane, 1,3-dioxane, 3,3-dimethyl-2-butanone, 4-methyl-2-pentanone, diethylcarbonate, n-butylacetate, 2-hexanone, 5-hexen-2-one, 1-ethoxy-2-propanol, hexanal, 2-(dimethylamino)-ethanol, 2-methylpyrazine, 1-methylpiperazine, valeronitrile, 4-methyl-2-hexanone, 1-methoxy-2-acetoxypropane, 2,6-dimethylmorpholine, methylhexanoate, 1-propoxy-2-propanol.

## Patentansprüche

1. Verfahren zur Reinigung von 2,3,3,3-Tetrafluor-1-propen (1234yf) ausgehend von einer ersten Zusammensetzung, die 2,3,3,3-Tetrafluor-1-propen, 3,3,3-Trifluorpropen (1243zf), trans-1,3,3,3-Tetrafluor-1-propen (1234ze-E) und gegebenenfalls oder nicht mindestens eine Verbindung aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) umfasst, wobei das Verfahren folgende Schritte umfasst:
a) Inkontaktbringen der ersten Zusammensetzung mit mindestens einem organischen Extraktionsmittel zur Bildung einer zweiten Zusammensetzung;
b) Extraktivdestillation der zweiten Zusammensetzung zur Bildung:
i) einer dritten Zusammensetzung, die das organische Extraktionsmittel, 3,3,3-Trifluorpropen (1243zf), trans-1,3,3,3-Tetrafluor-1-propen (1234ze-E) und gegebenenfalls oder nicht mindestens eine Verbindung aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) umfasst; und
ii) eines Stroms, der 2,3,3,3-Tetrafluor-1-propen umfasst;
c) Gewinnung und Trennung der dritten Zusammensetzung zur Bildung eines Stroms, der das organische Extraktionsmittel umfasst, und eines Stroms, der 3,3,3-Trifluorpropen (1243zf), trans-1,3,3,3-Tetrafluor-1-propen (1234ze-E) und gegebenenfalls oder nicht mindestens eine Verbindung aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) umfasst; wobei der Strom, der das organische Extraktionsmittel umfasst, vorzugsweise zu Schritt a) zurückgeführt wird;
**dadurch gekennzeichnet, dass** das organische Extraktionsmittel aus der Gruppe bestehend aus Acetaldehyd, Methylformiat, 2-Methoxy-1-propen, 1,2-Epoxypropan, Ethoxyethen, Dimethoxymethan, 1-Chlor-1,2,2-trifluorpropan, 3-Chlor-1,1,1-trifluorpropan, Ethandial, 2-Chlor-1,1,1,3-tetrafluorpropan, Propanon, Methylacetat, 4-Methoxy-2-methyl-2-butanthiol, Difluordiethylsilan, Isobutanal, Isopropylformiat, Methylglyoxal, 2,3-Dichlor-1,1,1-trifluorpropan, Ethylacetat, Butanon, n-Propylformiat, 1,3-Dichlor-1,1,2-trifluorpropan, 1,1-Dichlor-2,2,3-trifluorpropan, 1,2-Dimethoxyethan, 1,3-Dichlor-1,2,2-trifluorpropan, Isopropylacetat, Diethylsulfid, 1,3-Dichlor-1,2,3-trifluorpropan, 2-Methylbutanal, 2-Allyloxyethanol, Ethylpropionat, 1,2-Dimethoxypropan, Dioxan, 3-Pentanon, 2-Pentanon, Trimethoxymethan, 1,3-Dioxan, 3,3-Dimethyl-2-butanon, Pyridin, n-Methylmorpholin, 4-Methyl-2-pentanon, Butyronitril, 1-Methoxy-2-propanol, Diethylcarbonat, n-Butylacetat, 2-Hexanon, 5-Hexen-2-on, 2-Methoxy-1-propanol, 1-Ethoxy-2-propanol, Hexanal, 2-(Dimethylamino)ethanol, 2-Methylpyrazin, 2-Ethoxy-1-propanol, 1-Methylpiperazin, n-Ethylmorpholin, Valeronitril, 4-Methyl-2-hexanon, 1-Methoxy-2-acetoxypropan, 2,6-Dimethylmorpholin, Methylhexanoat, 2-Propoxyethanol und 1-Propoxy-2-propanol ausgewählt wird.

2. Verfahren nach einem der vorhergehenden Ansprüche, **dadurch gekennzeichnet, dass** es sich bei der ersten Zusammensetzung um eine azeotrope oder quasiazeotrope Zusammensetzung handelt, die 2,3,3,3-Tetrafluor-1-propen, trans-1,3,3,3-Tetrafluor-1-propen (1234ze-E), 3,3,3-Trifluorpropen (1243zf) und gegebenenfalls oder nicht mindestens eine der Verbindungen aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) umfasst.

3. Verfahren nach einem der vorhergehenden Ansprüche, **dadurch gekennzeichnet, dass** das organische Extraktionsmittel aus der Gruppe bestehend aus 2-Methoxy-1-propen, 1,2-Epoxypropan, Ethoxyethen, Dimethoxymethan, Methylacetat, Isobutanal, Isopropylformiat, Ethylacetat, Butanon, n-Propylformiat, 1,2-Dimethoxyethan, Isopropylacetat, 2-Methylbutanal, Ethylpropionat, 1,2-Dimethoxypropan, Dioxan, 3-Pentanon, 2-Pentanon, Trimethoxymethan, 1,3-Dioxan, 3,3-Dimethyl-2-butanon, 4-Methyl-2-pentanon, Diethylcarbonat, n-Butylacetat, 2-Hexanon, 5-Hexen-2-on, 1-Ethoxy-2-propanol, Hexanal, 2-(Dimethylamino)ethanol, 2-Methylpyrazin, 1-Methylpiperazin, Valeronitril, 4-Methyl-2-hexanon, 1-Methoxy-2-acetoxypropan, 2,6-Dimethylmorpholin, Methylhexanoat und 1-Propoxy-2-propanol ausgewählt wird; vorteilhafterweise das organische Extraktionsmittel aus der Gruppe bestehend aus Ethoxyethen, Dimethoxymethan, Methylacetat, Isobutanal, Isopropylformiat, Ethylacetat, Butanon, 1,2-Dimethoxyethan, Isopropylacetat, Dioxan, 3-Pentanon, 2-Pentanon, Trimethoxymethan, 1,3-Dioxan, 3,3-Dimethyl-2-butanon, 4-Methyl-2-pentanon, Diethylcarbonat, n-Butylacetat, 1-Ethoxy-2-propanol und Hexanal ausgewählt wird; vorzugsweise das organische Extraktionsmittel aus der Gruppe bestehend aus Dimethoxymethan, Butanon, Isopropylacetat, Dioxan, Trimethoxymethan, 1,3-Dioxan, n-Butylacetat, 1-Ethoxy-2-propanol und Hexanal ausgewählt wird; insbesondere das organische Extraktionsmittel aus der Gruppe bestehend aus Dimethoxymethan, Isopropylacetat, Dioxan, Trimethoxymethan, 1,3-Dioxan, n-Butylacetat, 1-Ethoxy-2-propanol und Hexanal ausgewählt wird.

4. Verfahren nach einem der vorhergehenden Ansprüche, **dadurch gekennzeichnet, dass** der in Schritt b) gebildete Strom, der 2,3,3,3-Tetrafluor-1-propen umfasst, gewonnen wird und frei von trans-1,3,3,3-Tetrafluor-1-propen (1234ze-E) und/oder von 3,3,3-Trifluorpropen (1243zf) und/oder von mindestens einer der Verbindungen aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) ist.

5. Verfahren nach einem der vorhergehenden Ansprüche, **dadurch gekennzeichnet, dass** die erste Zusammensetzung Verunreinigungen mit einem unter dem Siedepunkt von 2,3,3,3-Tetrafluor-1-propen liegenden Siedepunkt und gegebenenfalls schwere Verunreinigungen umfasst, wobei das Verfahren vor Schritt a) folgende Schritte umfasst:
i') Verwendung oder Bereitstellung einer Zusammensetzung, die 2,3,3,3-Tetrafluor-1-propen, Verunreinigungen mit einem unter dem Siedepunkt von 2,3,3,3-Tetrafluor-1-propen liegenden Siedepunkt, 3,3,3-Trifluorpropen (1243zf), trans-1,3,3,3-Tetrafluor-1-propen (1234ze-E) und gegebenenfalls oder nicht mindestens eine der Verbindungen aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) und gegebenenfalls schwere Verunreinigungen umfasst;
ii') Destillation der Zusammensetzung aus Schritt i) zur Entfernung von Verunreinigungen mit einem unter dem Siedepunkt von 2,3,3,3-Tetrafluor-1-propen liegenden Siedepunkt am Kopf der Säule und zur Bildung eines ersten Stroms, der 2,3,3,3-Tetrafluor-1-propen, 3,3,3-Trifluorpropen (1243zf), trans-1,3,3,3-Tetrafluor-1-propen (1234ze-E) und gegebenenfalls oder nicht mindestens eine der Verbindungen aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) und gegebenenfalls schwere Verunreinigungen umfasst und am Sumpf der Destillationssäule gewonnen wird;
iii') gegebenenfalls Destillation des am Sumpf der Destillationssäule in Schritt ii') gewonnenen ersten Stroms zur Gewinnung eines zweiten Stroms, der 2,3,3,3-Tetrafluor-1-propen, 3,3,3-Trifluorpropen (1243zf), trans-1,3,3,3-Tetrafluor-1-propen (1234ze-E) und gegebenenfalls oder nicht mindestens eine der Verbindungen aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) umfasst, am Kopf der Säule und eines Stroms, der die schweren Verunreinigungen umfasst, am Sumpf der Destillationssäule;
wobei der in Schritt ii') gewonnene erste Strom oder der in Schritt iii') gewonnene zweite Strom der in Schritt a) verwendeten ersten Zusammensetzung entspricht.

6. Verfahren zur Herstellung oder Reinigung von 2,3,3,3-Tetrafluor-1-propen, das folgende Schritte umfasst:
A) Fluorierung einer Verbindung der Formel (I) CX (Y)₂-CX(Y)ₘ-CHₘXY, in der X und Y unabhängig für ein Wasserstoff-, Fluor- oder Chloratom stehen und m = 0 oder 1, in Gegenwart eines Katalysators und/oder Fluorierung einer Verbindung der Formel (CXₙY₃₋ₙ)CHₚX₁₋ₚCHₘX₂₋ₘ (II), in der X unabhängig voneinander für Cl, F, I oder Br steht; Y unabhängig voneinander für H, Cl, F, I oder Br steht; n für 1, 2 oder 3 steht und m für 0, 1 oder 2 steht und p für 0 oder 1 steht, in Gegenwart eines Katalysators;
B) Gewinnung eines Stroms, der 2,3,3,3-Tetrafluor-1-propen, 3,3,3-Trifluorpropen (1243zf), trans-1,3,3,3-Tetrafluor-1-propen (1234ze-E) und gegebenenfalls oder nicht mindestens eine der Verbindungen aus der Gruppe bestehend aus Chlormethan (40), 1,1-Difluorethan (152a), Chlorpentafluorethan (115), 1,1,1,2-Tetrafluorethan (134a) und trans-1,2,3,3,3-Pentafluorpropen (1225ye-E) umfasst;
C) Durchführung des Verfahrens nach einem der Ansprüche 1 bis 5 unter Verwendung des in Schritt B) gewonnenen Stroms.

7. Zusammensetzung, umfassend 2,3,3,3-Tetrafluorpropen, 3,3,3-Trifluorpropen (1243zf), trans-1,3,3,3-Tetrafluor-1-propen (1234ze-E) und ein organisches Extraktionsmittel aus der Gruppe bestehend aus Acetaldehyd, Methylformiat, 2-Methoxy-1-propen, 1,2-Epoxypropan, Ethoxyethen, Dimethoxymethan, 1-Chlor-1,2,2-trifluorpropan, 3-Chlor-1,1,1-trifluorpropan, Ethandial, 2-Chlor-1,1,1,3-tetrafluorpropan, Propanon, Methylacetat, 4-Methoxy-2-methyl-2-butanthiol, Difluordiethylsilan, Isobutanal, Isopropylformiat, Methylglyoxal, 2,3-Dichlor-1,1,1-trifluorpropan, Ethylacetat, Butanon, n-Propylformiat, 1,3-Dichlor-1,1,2-trifluorpropan, 1,1-Dichlor-2,2,3-trifluorpropan, 1,2-Dimethoxyethan, 1,3-Dichlor-1,2,2-trifluor-propan, Isopropylacetat, Diethylsulfid, 1,3-Dichlor-1,2,3-trifluorpropan, 2-Methylbutanal, 2-Allyloxyethanol, Ethylpropionat, 1,2-Dimethoxypropan, Dioxan, 3-Pentanon, 2-Pentanon, Trimethoxymethan, 1,3-Dioxan, 3,3-Dimethyl-2-butanon, Pyridin, n-Methylmorpholin, 4-Methyl-2-pentanon, Butyronitril, 1-Methoxy-2-propanol, Diethylcarbonat, n-Butylacetat, 2-Hexanon, 5-Hexen-2-on, 2-Methoxy-1-propanol, 1-Ethoxy-2-propanol, Hexanal, 2-(Dimethylamino)ethanol, 2-Methylpyrazin, 2-Ethoxy-1-propanol, 1-Methylpiperazin, n-Ethylmorpholin, Valeronitril, 4-Methyl-2-hexanon, 1-Methoxy-2-acetoxypropan, 2,6-Dimethylmorpholin, Methylhexanoat, 2-Propoxyethanol und 1-Propoxy-2-propanol.

8. Zusammensetzung nach dem vorhergehenden Anspruch, **dadurch gekennzeichnet, dass** das organische Extraktionsmittel aus der Gruppe bestehend aus 2-Methoxy-1-propen, 1,2-Epoxypropan, Ethoxyethen, Dimethoxymethan, Methylacetat, Isobutanal, Isopropylformiat, Ethylacetat, Butanon, n-Propylformiat, 1,2-Dimethoxyethan, Isopropylacetat, 2-Methylbutanal, Ethylpropionat, 1,2-Dimethoxypropan, Dioxan, 3-Pentanon, 2-Pentanon, Trimethoxymethan, 1,3-Dioxan, 3,3-Dimethyl-2-butanon, 4-Methyl-2-pentanon, Diethylcarbonat, n-Butylacetat, 2-Hexanon, 5-Hexen-2-on, 1-Ethoxy-2-propanol, Hexanal, 2-(Dimethylamino)ethanol, 2-Methylpyrazin, 1-Methylpiperazin, Valeronitril, 4-Methyl-2-hexanon, 1-Methoxy-2-acetoxypropan, 2,6-Dimethylmorpholin, Methylhexanoat und 1-Propoxy-2-propanol ausgewählt ist.

## Claims

1. Process for purifying 2,3,3,3-tetrafluoro-1-propene (1234yf) using a first composition comprising 2,3,3,3-tetrafluoro-1-propene, 3,3,3-trifluoropropene (1243zf), trans-1,3,3,3-tetrafluoro-1-propene (1234ze-E) and optionally or not at least one compound chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E), said process comprising the steps of:
a) placing said first composition in contact with at least one organic extracting agent to form a second composition;
b) extractive distillation of said second composition to form:
i) a third composition comprising said organic extracting agent, 3,3,3-trifluoropropene (1243zf), trans-1,3,3,3-tetrafluoro-1-propene (1234ze-E) and optionally or not at least one compound chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E); and
ii) a stream comprising 2,3,3,3-tetrafluoro-1-propene;
c) recovery and separation of said third composition to form a stream comprising said organic extracting agent and a stream comprising 3,3,3-trifluoropropene (1243zf), trans-1,3,3,3-tetrafluoro-1-propene (1234ze-E) and optionally or not at least one compound chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E); preferably, the stream comprising said organic extracting agent is recycled into step a);
**characterized in that** said organic extracting agent is chosen from the group consisting of acetaldehyde, methyl formate, 2-methoxy-1-propene, 1,2-epoxypropane, ethoxyethene, dimethoxymethane, 1-chloro-1,2,2-trifluoropropane, 3-chloro-1,1,1-trifluoropropane, ethanedial, 2-chloro-1,1,1,3-tetrafluoropropane, propanone, methyl acetate, 4-methoxy-2-methyl-2-butanethiol, difluorodiethylsilane, isobutanal, isopropyl formate, methylglyoxal, 2,3-dichloro-1,1,1-trifluoropropane, ethyl acetate, butanone, n-propyl formate, 1,3-dichloro-1,1,2-trifluoropropane, 1,1-dichloro-2,2,3-trifluoropropane, 1,2-dimethoxyethane, 1,3-dichloro-1,2,2-trifluoropropane, isopropyl acetate, diethyl sulfide, 1,3-dichloro-1,2,3-trifluoropropane, 2-methylbutanal, 2-allyloxyethanol, ethyl propionate, 1,2-dimethoxypropane, dioxane, 3-pentanone, 2-pentanone, trimethoxymethane, 1,3-dioxane, 3,3-dimethyl-2-butanone, pyridine, n-methylmorpholine, 4-methyl-2-pentanone, butyronitrile, 1-methoxy-2-propanol, diethyl carbonate, n-butyl acetate, 2-hexanone, 5-hexen-2-one, 2-methoxy-1-propanol, 1-ethoxy-2-propanol, hexanal, 2-(dimethylamino)ethanol, 2-methylpyrazine, 2-ethoxy-1-propanol, 1-methylpiperazine, n-ethylmorpholine, valeronitrile, 4-methyl-2-hexanone, 1-methoxy-2-acetoxypropane, 2,6-dimethylmorpholine, methyl hexanoate, 2-propoxyethanol and 1-propoxy-2-propanol.

2. Process according to any one of the preceding claims, **characterized in that** said first composition is an azeotropic or quasi-azeotropic composition comprising 2,3,3,3-tetrafluoro-1-propene, trans-1,3,3,3-tetrafluoro-1-propene (1234ze-E), 3,3,3-trifluoropropene (1243zf) and optionally or not at least one compound chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E).

3. Process according to any one of the preceding claims, **characterized in that** said organic extracting agent is chosen from the group consisting of 2-methoxy-1-propene, 1,2-epoxypropane, ethoxyethene, dimethoxymethane, methyl acetate, isobutanal, isopropyl formate, ethyl acetate, butanone, n-propyl formate, 1,2-dimethoxyethane, isopropyl acetate, 2-methylbutanal, ethyl propionate, 1,2-dimethoxypropane, dioxane, 3-pentanone, 2-pentanone, trimethoxymethane, 1,3-dioxane, 3,3-dimethyl-2-butanone, 4-methyl-2-pentanone, diethyl carbonate, n-butyl acetate, 2-hexanone, 5-hexen-2-one, 1-ethoxy-2-propanol, hexanal, 2-(dimethylamino)ethanol, 2-methylpyrazine, 1-methylpiperazine, valeronitrile, 4-methyl-2-hexanone, 1-methoxy-2-acetoxypropane, 2,6-dimethylmorpholine, methyl hexanoate, and 1-propoxy-2-propanol; advantageously, said organic extracting agent is chosen from the group consisting of ethoxyethene, dimethoxymethane, methyl acetate, isobutanal, isopropyl formate, ethyl acetate, butanone, 1,2-dimethoxyethane, isopropyl acetate, dioxane, 3-pentanone, 2-pentanone, trimethoxymethane, 1,3-dioxane, 3,3-dimethyl-2-butanone, 4-methyl-2-pentanone, diethyl carbonate, n-butyl acetate, 1-ethoxy-2-propanol and hexanal; preferably, said organic extracting agent is chosen from the group consisting of dimethoxymethane, butanone, isopropyl acetate, dioxane, trimethoxymethane, 1,3-dioxane, n-butyl acetate, 1-ethoxy-2-propanol and hexanal; in particular, said organic extracting agent is chosen from the group consisting of dimethoxymethane, isopropyl acetate, dioxane, trimethoxymethane, 1,3-dioxane, n-butyl acetate, 1-etho>cy-2-propanol and hexanal.

4. Process according to any one of the preceding claims, **characterized in that** the stream comprising 2,3,3,3-tetrafluoro-1-propene formed in step b) is recovered and is free of trans-1,3,3,3-tetrafluoro-1-propene (1234ze-E) and/or of 3,3,3-trifluoropropene (1243zf) and/or of at least one of the compounds chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E).

5. Process according to any one of the preceding claims, **characterized in that** said first composition comprises impurities with a boiling point below the boiling point of 2,3,3,3-tetrafluoro-1-propene and optionally heavy impurities, and the process comprising, prior to step a), the following steps:
i') use or provision of a composition comprising 2,3,3,3-tetrafluoro-1-propene, impurities with a boiling point below the boiling point of 2,3,3,3-tetrafluoro-1-propene, 3,3,3-trifluoropropene (1243zf), trans-1,3,3,3-tetrafluoro-1-propene (1234ze-E) and optionally or not at least one compound chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E), and optionally heavy impurities;
ii') distillation of said composition from step i) to remove, at the top of the column, impurities with a boiling point below the boiling point of 2,3,3,3-tetrafluoro-1-propene and to form a first stream comprising 2,3,3,3-tetrafluoro-1-propene, 3,3,3-trifluoropropene (1243zf), trans-1,3,3,3-tetrafluoro-1-propene (1234ze-E) and optionally or not at least one compound chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E), and optionally heavy impurities; recovered at the bottom of the distillation column;
iii') optionally, distillation of said first stream recovered at the bottom of the distillation column in step ii') to recover, at the top of the column, a second stream comprising 2,3,3,3-tetrafluoro-1-propene, 3,3,3-trifluoropropene (1243zf), trans-1,3,3,3-tetrafluoro-1-propene (1234ze-E) and optionally or not at least one compound chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E), and, at the bottom of the distillation column, a stream comprising the heavy impurities;
said first stream recovered in step ii') or said second stream recovered in step iii') corresponding to said first composition used in step a).

6. Process for producing and purifying 2,3,3,3-tetrafluoro-1-propene, comprising the steps of:
A) fluorination, in the presence of a catalyst, of a compound of formula (I) CX(Y)₂-CX(Y)ₘ-CHₘXY in which X and Y independently represent a hydrogen, fluorine or chlorine atom and m = 0 or 1 and/or fluorination, in the presence of a catalyst, of a compound of formula (CXₙY₃₋ₙ)CHₚX₁₋ₚCHₘX₂₋ₘ (II) in which X is, independently of each other, Cl, F, I or Br; Y is, independently of each other, H, Cl, F, I or Br; n is 1, 2 or 3; and m is 0, 1 or 2; and p is 0 or 1;
B) recovery of a stream comprising 2,3,3,3-tetrafluoro-1-propene, 3,3,3-trifluoropropene (1243zf), trans-1,3,3,3-tetrafluoro-1-propene (1234ze-E) and optionally or not at least one compound chosen from the group consisting of chloromethane (40), 1,1-difluoroethane (152a), chloropentafluoroethane (115), 1,1,1,2-tetrafluoroethane (134a) and trans-1,2,3,3,3-pentafluoropropene (1225ye-E);
C) implementation of the process according to any one of Claims 1 to 5 using the stream recovered in step B).

7. Composition comprising 2,3,3,3-tetrafluoropropene, 3,3,3-trifluoropropene (1243zf), trans-1,3,3,3-tetrafluoro-1-propene (1234ze-E) and an organic extracting agent chosen from the group consisting of acetaldehyde, methyl formate, 2-methoxy-1-propene, 1,2-epoxypropane, ethoxyethene, dimethoxymethane, 1-chloro-1,2,2-trifluoropropane, 3-chloro-1,1,1-trifluoropropane, ethanedial, 2-chloro-1,1,1,3-tetrafluoropropane, propanone, methyl acetate, 4-methoxy-2-methyl-2-butanethiol, difluorodiethylsilane, isobutanal, isopropyl formate, methylglyoxal, 2,3-dichloro-1,1,1-trifluoropropane, ethyl acetate, butanone, n-propyl formate, 1,3-dichloro-1,1,2-trifluoropropane, 1,1-dichloro-2,2,3-trifluoropropane, 1,2-dimethoxyethane, 1,3-dichloro-1,2,2-trifluoropropane, isopropyl acetate, diethyl sulfide, 1,3-dichloro-1,2,3-trifluoropropane, 2-methylbutanal, 2-allyloxyethanol, ethyl propionate, 1,2-dimethoxypropane, dioxane, 3-pentanone, 2-pentanone, trimethoxymethane, 1,3-dioxane, 3,3-dimethyl-2-butanone, pyridine, n-methylmorpholine, 4-methyl-2-pentanone, butyronitrile, 1-methoxy-2-propanol, diethyl carbonate, n-butyl acetate, 2-hexanone, 5-hexen-2-one, 2-methoxy-1-propanol, 1-ethoxy-2-propanol, hexanal, 2-(dimethylamino)ethanol, 2-methylpyrazine, 2-ethoxy-1-propanol, 1-methylpiperazine, n-ethylmorpholine, valeronitrile, 4-methyl-2-hexanone, 1-methoxy-2-acetoxypropane, 2,6-dimethylmorpholine, methyl hexanoate, 2-propoxyethanol and 1-propoxy-2-propanol.

8. Composition according to the preceding claim, **characterized in that** said organic extracting agent is chosen from the group consisting of 2-methoxy-1-propene, 1,2-epoxypropane, ethoxyethene, dimethoxymethane, methyl acetate, isobutanal, isopropyl formate, ethyl acetate, butanone, n-propyl formate, 1,2-dimethoxyethane, isopropyl acetate, 2-methylbutanal, ethyl propionate, 1,2-dimethoxypropane, dioxane, 3-pentanone, 2-pentanone, trimethoxymethane, 1,3-dioxane, 3,3-dimethyl-2-butanone, 4-methyl-2-pentanone, diethyl carbonate, n-butyl acetate, 2-hexanone, 5-hexen-2-one, 1-ethoxy-2-propanol, hexanal, 2-(dimethylamino)ethanol, 2-methylpyrazine, 1-methylpiperazine, valeronitrile, 4-methyl-2-hexanone, 1-methoxy-2-acetoxypropane, 2,6-dimethylmorpholine, methyl hexanoate and 1-propoxy-2-propanol.
